(19) Europäisches Patentamt / European Patent Office / Office européen des brevets

(11) **EP 4 397 679 A1**

(12) **EUROPEAN PATENT APPLICATION**

(43) Date of publication:
**10.07.2024 Bulletin 2024/28**

(21) Application number: **24150099.0**

(22) Date of filing: **02.01.2024**

(51) International Patent Classification (IPC):
**C07K 14/71** (2006.01)    **C07K 14/495** (2006.01)
**A61K 38/17** (2006.01)

(52) Cooperative Patent Classification (CPC):
**C07K 14/71; A61K 38/179; C07K 14/495;**
C07K 2319/30

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **03.01.2023 US 202363436822 P**

(71) Applicant: **FBD Biologics Limited
Kowloon (HK)**

(72) Inventors:
• **WANG, Jiin-Tarng
Taichung City, 411013 (CN)**
• **KUO, Pan-Hsien
Taoyuan City, 335004 (CN)**
• **JUO, Zong SEAN
New Taipei, 231 (CN)**
• **TSENG, Chi-Ling
Taipei City, 115 (CN)**

(74) Representative: **Fish & Richardson P.C.
Highlight Business Towers
Mies-van-der-Rohe-Straße 8
80807 München (DE)**
Remarks:
•The complete document including Reference
Table(s) and the Sequence Listing(s) can be
downloaded from the EPO website
•Claims filed after the date of filing of the application
(Rule 68(4) EPC).

(54) **ENGINEERED TGFBRII VARIANTS AND METHODS OF USE THEREOF**

(57)    This disclosure relates to engineered TGFβRII variants, protein constructs, and methods of use thereof.

**FIG. 1B**

## Description

## CROSS-REFERENCE TO RELATED APPLICATION

This disclosure claims priority to and benefit of U.S. Provisional Patent Application Serial No 63/436,822, filed January 03, 2023, which is incorporated herein by reference in its entirety.

## SEQUENCE LISTING

This application contains a Sequence Listing that has been submitted electronically as an XML file named 52246-0011EP1_SL_ST26.xml. The XML file, created on October 4, 2023, is 19,331 bytes in size. The material in the XML file is hereby incorporated by reference in its entirety.

## TECHNICAL FIELD

This disclosure relates to engineered TGFβRII variants, and methods of use thereof.

## BACKGROUND

TGF-beta receptor type-2 (TGFBR2, TGFβRII, or TβRII) is the ligand-binding receptor for all members of the TGF-β family and expressed in virtually all cell types including fibroblasts.

The transforming growth factor (TGF) β signaling pathway is involved in many cellular processes including proliferation, differentiation, adhesion, motility and apoptosis. These functions are frequently disrupted in malignant cells and the TGFβ type II receptor (TGFβRII) was subsequently demonstrated to be a tumor suppressor gene (TSG). The TGFβ signaling pathway mediates potent growth inhibition in normal cells and in line with its role as a TSG, cancer cells use both genetic and epigenetic mechanisms to inactivate TGFβRII.

Considering its roles in both normal cells and cancer cells, there is a need to develop cancer therapies targeting TGFβRII pathway with limited toxicities.

## SUMMARY

This disclosure relates to engineered TGFβRII variants, and methods of use thereof.

In one aspect, the disclosure is related to an engineered TGFβRII polypeptide comprising an amino acid sequence that is at least 80% identical to SEQ ID NO: 7, wherein the engineered TGFβRII polypeptide comprises one or more amino acid mutations at positions corresponding to S49 and D118 of SEQ ID NO: 7.

In some embodiments, the engineered TGFβRII polypeptide comprises one or more of the following:

(a) the amino acid that corresponds to S49 of SEQ ID NO: 7 is G, A, L, M, F, W, K, Q, E, P, V, I, C, Y, H, R, N, D, or T; and
(b) the amino acid that corresponds to D118 of SEQ ID NO: 7 is G, A, L, M, F, W, K, Q, E, S, P, V, I, C, Y, H, R, N, or T.

In some embodiments, the amino acid that corresponds to S49 of SEQ ID NO: 7 is E or Q.

In some embodiments, the amino acid that corresponds to D118 of SEQ ID NO: 7 is E.

In some embodiments, the amino acid that corresponds to S49 of SEQ ID NO: 7 is E.

In some embodiments, the amino acid that corresponds to position 32 of SEQ ID NO: 7 is D.

In some embodiments, the amino acid that corresponds to position 119 of SEQ ID NO: 7 is E.

In some embodiments, wherein the engineered TGFβRII polypeptide comprises an amino acid sequence that is at least 85%, 90%, 95%, 95%, 96%, 97%, 98%, 99%, or 100% identical to any one of SEQ ID NOs: 7-12.

In some embodiments, the engineered TGFβRII polypeptide comprises an amino acid sequence that is at least 90% identical to SEQ ID NO: 8.

In some embodiments, the engineered TGFβRII polypeptide comprises an amino acid sequence that is at least 90% identical to SEQ ID NO: 9.

In some embodiments, the engineered TGFβRII polypeptide comprises an amino acid sequence that is at least 90% identical to SEQ ID NO: 10.

In some embodiments, the engineered TGFβRII polypeptide comprises an amino acid sequence that is at least 90% identical to SEQ ID NO: 11.

In some embodiments, the engineered TGFβRII polypeptide comprises an amino acid sequence that is at least 90% identical to SEQ ID NO: 12.

In some embodiments, the off rate between the engineered TGFβRII polypeptide and TGFβ1 is lower than the off rate between wildtype TGFβRII and TGFβ1.

In some embodiments, the off rate between the engineered TGFβRII polypeptide and TGFβ3 is lower than the off rate between wildtype TGFβRII and TGFβ3.

In some embodiments, the engineered TGFβRII polypeptide does not bind to TGFβ2.

In some embodiments, the engineered TGFβRII polypeptide interacts with R94, R25, K37, and/or K31 on TGFβ1.

In some embodiments, the engineered TGFβRII polypeptide interacts with R94, R25, K37, and/or K31 on TGFβ3.

In some embodiments, the engineered TGFβRII polypeptide interacts with R94, R25, K37, and/or K31 on TGFβ1 via hydrogen bond interactions.

In some embodiments, the engineered TGFβRII polypeptide interacts with R94, R25, K37, and/or K31 on TGFβ3 via hydrogen bond interactions.

In some embodiments, the engineered TGFβRII polypeptide can capture TGFβ (e.g., TGFβ1) and thereby improve tumor microenvironment.

In some embodiments, the engineered TGF-

βRII polypeptide can block or interfere the binding between TGFβ (e.g., TGFβ1) and TGFβRII.

In some embodiments, the engineered TGF-βRII polypeptide can block or interfere the binding between TGFβ (e.g., TGFβ1) and TGFβRII expressed on the surface of a tumor cells, fibroblasts, or immune cells.

In some embodiments, the engineered TGF-βRII polypeptide further comprises a CH2 domain and a CH3 domain.

In some embodiments, the engineered TGF-βRII polypeptide further comprises a hinge region.

In some embodiments, the CH2 domain is an IgG CH2 domain and the CH3 domain is an IgG CH3 domain.

In some embodiments, the engineered TGF-βRII polypeptide are linked to either (a) the N-terminus of the hinge region or CH2 domain, or (b) the C-terminus of the CH3 domain, optionally through a linker sequence.

In some embodiments, the engineered TGF-βRII polypeptide comprises an amino acid sequence that is identical to any one of SEQ ID NO: 1-6.

In one aspect, the disclosure is related to a protein construct comprising the engineered TGFβRII polypeptide described herein.

In some embodiments, the protein construct comprises two or more engineered TGFβRII polypeptides.

In some embodiments, at least two of the engineered TGFβRII polypeptides are identical.

In some embodiments, at least two of the engineered TGFβRII polypeptides are different.

In some embodiments, the protein construct further comprises an Fc region.

In some embodiments, the Fc region is an IgG4 Fc region.

T In some embodiments, the Fc region is an IgG1 Fc region (e.g., with LALA mutations or LALA-PG mutations).

In some embodiments, the engineered TGF-βRII polypeptide is linked to the C-terminus of the Fc region.

In some embodiments, the engineered TGF-βRII polypeptide is linked to the C-terminus of the Fc region via a peptide linker.

In one aspect, the disclosure is related to a protein construct comprising a first fusion polypeptide comprising the engineered TGFβRII polypeptide described herein, a first CH2 domain, and a first CH3 domain; and a second fusion polypeptide comprising a second CH2 domain, and a second CH3 domain, wherein the first fusion polypeptide and the second fusion polypeptide associate with each other, forming a dimer.

In some embodiments, the second fusion polypeptide further comprises a second engineered TGFβRII polypeptide.

In one aspect, the disclosure is related to a pharmaceutical composition comprising the engineered TGFβRII polypeptide described herein or the protein construct described herein; and a pharmaceutically acceptable carrier.

In one aspect, the disclosure is related to a nucleic acid encoding the engineered TGFβRII polypeptide described herein or the protein construct described herein.

In one aspect, the disclosure is related to a vector comprising the nucleic acid described herein.

In one aspect, the disclosure is related to a cell comprising the nucleic acid described herein or the vector described herein.

In some embodiments, the cell is a CHO cell.

In one aspect, the disclosure is related to a method of producing an engineered TGFβRII polypeptide or a protein construct comprising the engineered TGFβRII polypeptide, the method comprising

(a) culturing the cell described herein under conditions sufficient for the cell to produce the engineered TGFβRII polypeptide or the protein construct; and
(b) collecting the engineered TGFβRII polypeptide or the protein construct produced by the cell.

In one aspect, the disclosure is related to a method of treating a subject having cancer, the method comprising administering a therapeutically effective amount of a composition comprising the engineered TGFβRII polypeptide described herein or the protein construct described herein to the subject.

In some embodiments, the subject has a solid tumor or a hematologic cancer.

In some embodiments, the cancer is breast cancer, ovarian cancer, glioma, colon cancer, hepatocellular carcinoma, prostate cancer, renal cell carcinoma, melanoma, lung cancer, pancreatic cancer, or hepatoma.

In one aspect, the disclosure is related to a method of decreasing the rate of tumor growth, the method comprising contacting a tumor cell with an effective amount of a composition comprising the engineered TGFβRII polypeptide described herein or the protein construct described herein.

In one aspect, the disclosure is related to a method of killing a tumor cell, the method comprising contacting a tumor cell with an effective amount of a composition comprising the engineered TGFβRII polypeptide described herein or the protein construct described herein.

As used herein, the term "engineered TGFβRII polypeptide" refers to a polypeptide derived from a wildtype TGFβRII polypeptide or a portion thereof (e.g., the extracellular region of TGFβRII) with one or more mutations (e.g., insertions, deletions, or substitutions). In some embodiments, the engineered TGFβRII polypeptide comprises or consists of the extracellular region of TGFβRII or a portion of the extracellular region.

As used herein, the term "protein construct" refers to a complex having one or more polypeptides. In some embodiments, the protein construct has two or more polypeptides, wherein the polypeptides can associate with each other, forming a dimer or a multimer.

As used herein, the term "cancer" refers to cells having the capacity for uncontrolled autonomous growth. Examples of such cells include cells having an abnormal state or condition characterized by rapidly proliferating cell growth. The term is meant to include cancerous growths, e.g., tumors; oncogenic processes, metastatic tissues, and malignantly transformed cells, tissues, or organs, irrespective of histopathologic type or stage of invasiveness. Also included are malignancies of the various organ systems, such as respiratory, cardiovascular, renal, reproductive, hematological, neurological, hepatic, gastrointestinal, and endocrine systems; as well as adenocarcinomas which include malignancies such as most colon cancers, renal-cell carcinoma, prostate cancer and/or testicular tumors, non-small cell carcinoma of the lung, and cancer of the small intestine. Cancer that is "naturally arising" includes any cancer that is not experimentally induced by implantation of cancer cells into a subject, and includes, for example, spontaneously arising cancer, cancer caused by exposure of a patient to a carcinogen(s), cancer resulting from insertion of a transgenic oncogene or knockout of a tumor suppressor gene, and cancer caused by infections, e.g., viral infections. The term "carcinoma" is art recognized and refers to malignancies of epithelial or endocrine tissues. The term also includes carcinosarcomas, which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation. The term "hematopoietic neoplastic disorders" includes diseases involving hyperplastic/neoplastic cells of hematopoietic origin. A hematopoietic neoplastic disorder can arise from myeloid, lymphoid or erythroid lineages, or precursor cells thereof. A hematologic cancer is a cancer that begins in blood-forming tissue, such as the bone marrow, or in the cells of the immune system. Examples of hematologic cancer include e.g., leukemia, lymphoma, and multiple myeloma etc.

As used herein, the terms "subject" and "patient" are used interchangeably throughout the specification and describe an animal, human or non-human, to whom treatment according to the methods of the present invention is provided. Veterinary and non-veterinary applications are contemplated in the present disclosure. Human patients can be adult humans or juvenile humans (e.g., humans below the age of 18 years old). In addition to humans, patients include but are not limited to mice, rats, hamsters, guinea-pigs, rabbits, ferrets, cats, dogs, and primates. Included are, for example, non-human primates (e.g., monkey, chimpanzee, gorilla, and the like), rodents (e.g., rats, mice, gerbils, hamsters, ferrets, rabbits), lagomorphs, swine (e.g., pig, miniature pig), equine, canine, feline, bovine, and other domestic, farm, and zoo animals.

As used herein, the terms "polypeptide," "peptide," and "protein" are used interchangeably to refer to polymers of amino acids of any length of at least two amino acids.

As used herein, the terms "polynucleotide," "nucleic acid molecule," and "nucleic acid sequence" are used interchangeably herein to refer to polymers of nucleotides of any length of at least two nucleotides, and include, without limitation, DNA, RNA, DNA/RNA hybrids, and modifications thereof.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

## DESCRIPTION OF DRAWINGS

FIG. 1A shows the 3D structure of the interface between TGFβRII extracellular domain and its ligand TGFβ1.

FIG. 1B shows the 3D structure of the interface between a mutated TGFβRII extracellular domain (mutations at S49 and D118) and its ligand TGFβ1.

FIG. 2 shows amino acid sequences of G4_TGFβRII proteins.

FIG. 3 shows a summary of production and CMC analysis results.

FIGS. 4A-4C show the binding affinities between G4_TGFβRII proteins and human TGFβ, as measured by ELISA.

FIG. 5A shows the on rate binding ability between G4_TGFβRII proteins and human TGFβ, as measured by ELISA.

FIG. 5B shows the off rate binding ability between G4_TGFβRII proteins and human TGFβ, as measured by ELISA.

FIG. 5C shows the EC50 for on rate binding ability between G4_TGFβRII proteins and human TGFβ, as measured by ELISA .

FIG. 6 shows the results of the BLI binding assay.

FIGs. 7A-7C show the results from the smad2 reporter activity assay.

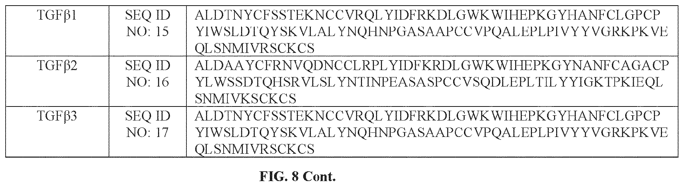

**FIG. 8** lists relevant protein sequences discussed in the disclosure.

## DETAILED DESCRIPTION

The transforming growth factor (TGF) β signaling pathway is involved in many cellular processes including proliferation, differentiation, adhesion, motility and apoptosis. These functions are frequently disrupted in malignant cells and the TGFβ type II receptor (TGFβRII) was subsequently demonstrated to be a tumor suppressor gene (TSG). The TGFβ signaling pathway mediates potent growth inhibition in normal cells and in line with its role as a TSG, cancer cells use both genetic and epigenetic mechanisms to inactivate TGFβRII. However, the relationship between the TGFβ signaling pathway and cancer progression is complex. The abrogation of TGFβ signaling provides a growth advantage to early stage malignant tumors but TGFβ assumes a pro-metastatic role in progressed tumors. Therefore, the loss of TGFβRII is associated with poor clinical outcome and is a predictor of poor prognosis in early stage breast cancer but over expression of the TGFβ ligand is associated with the metastatic phenotype in many tumors. This dual nature of TGFβ presents a challenge with respect to restoring TGFβ signaling to take advantage of the growth suppressive effects.

In the mammalian system, TGFβs are classified as TGFβ1, TGFβ2, and TGFβ3 which are encoded by different genes but they all function through the same receptor signaling system. TGFβ is secreted as a latent complex bound to other extracellular proteins like latent TGFβ binding proteins that tether the TGFβ in the extracellular matrix. TGFβ binds to TGFβRIII at the cell surface which presents the ligand to the TGFβRII receptors. The intracellular signaling is initiated upon the selective binding of the active cytokine to the TGFβRII homodimer which has constitutively active Ser/Thr kinase activity. Upon TGFβ binding, TGFβRII forms a heterotetramer with TGFβRI comprising of two identical TGFβRI/TGFβRII receptor heterodimers bound to dimeric TGFβ. Once the receptor complex is formed, TGFβRII transphosphorylates and activates the TGFβRI Ser/Thr kinase. Activation of TGFβRI propagates downstream signaling via the Smad family of proteins. The TGFβRI receptor directly interacts with and phosphorylates Smad 2 and Smad 3 (also termed as receptor activated Smads or R-Smads). These Smads bind to Smad 4 (also termed Co-Smad) which results in the translocation of this complex to the nucleus where the Smads regulate TGFβ-responsive gene expression. Systemic administration of TGFβ receptor trap can inhibit tumor cell proliferation. It suggests that TGFβ acts as a promoter rather than a suppressor in the relatively early stages of tumorigenesis.

A detailed description of TGFβ, TGFBR2, and the use of TGFβ trap to treat cancers are described, e.g., in Chowdhury, Sanjib, Sudhakar Ammanamanchi, and Gillian M. Howell. "Epigenetic targeting of transforming growth factor β receptor II and implications for cancer therapy." Molecular and cellular pharmacology 1.1 (2009): 57; Bierie, B., et al. "TGFβ: the molecular Jekyll and Hyde of cancer." Nature Reviews Cancer 6.7 (2006): 506-520; Kim, B., et al. "Novel therapies emerging in oncology to target the TGF-β pathway." Journal of Hematology & Oncology 14.1 (2021): 1-20; and Lind, H., et al. "Dual targeting of TGF-β and PD-L1 via a bifunctional anti-PD-L1/TGF-βRII agent: status of preclinical and clinical advances." Journal for immunotherapy of cancer 8.1 (2020); each of which is incorporated by reference in its entirety.

The present disclosure provides engineered TGFβRII variants. These engineered TGFβRII variants can be used to target TGFβRII pathway, whereas the interaction of engineered TGFβRII variants and TGFβ are carefully modulated.

## Engineered TGFβRII variants

TGFβ family ligands initiate signaling through the assembly of a receptor complex consisting of a ligand, two type I, and two type II receptors. Each receptor ECD consists of ~100 residues exhibiting a three-finger toxin fold of three β-strands. Both type I and type II receptors display a high degree of homology, except for the extended loops that flank the core β-sheet. Four of the five type II and four of the seven type I receptor ECDs have been structurally characterized either alone (type II: ActRIIA, TBRII, BMPII/type I: Alk1, Alk3, Alk5) or in a ligand-receptor complex (type II: ActRIIA, ActRIIB, TBRII, BMPII/type I: Alk1, Alk3, Alk5, Alk6). These structures have revealed a common set of four disulfide bonds that stabilize the β-sheet structure in each receptor. Additionally, ActRIIA, ActRIIB, BMPRII, and the type I receptors contain an additional disulfide bond to stabilize the β4-β5 loop and preventing the occlusion of the surface of the receptor, a crucial region for maintaining ligand binding, particularly for the type II receptors. TGFβRII is unique in having two additional disulfide bonds to tether the β1-β2 loop in a position for ligand interaction.

TGFβRII is highly specific for binding to the TGFβ class (TGFβ1-3). During complex formation with TGFβ, TGFβRII binds at the distal fingertips of the ligand, where the receptor β-sheet is perpendicular to the β-strands of the fingers. Specifically, β2 of TGFβRII forms significant contacts and binds within a cleft formed from positively charged residues (Arg303 and Arg372 in TGFβ1)-a feature shared by the TGFβ ligands. These residues account for TGFβ ligand specificity for TGFβRII and are not present in BMP or activin class ligands. This interaction is further stabilized by an extension in the β1-β2 loop that is limited to TGFβRII.

The TGF-β type II receptor, TGFβRII, is unique among the type I and type II receptors of the family in that it binds its cognate ligands through a distinct interface, namely, an edge β-strand on the smaller β1-β2-sheet, the β2-strand, and the flanking β1 and β1' strands.

Use of this alternative interface is promoted by two important structural differences in TGFβRII compared with ActRII, ActRIIB, and BMPRII. First, the β4-β5 loop is extended by seven to eight residues in TGFβRII relative to other type II receptors and lacks the f disulfide. The absence of this disulfide allows the extended β4-β5 loop to fold onto the concave surface of the β4-β3-β5 sheet, thus blocking ligand binding through this interface. Second, the β1-β2 loop in TGFβRII is extended by more than 10 residues compared with all other type I and type II receptors of the family, except AMHRII. The β1-β2 loop is further stabilized by both the e disulfide at its base, as in type I receptors, and the TGFβRII-specific g disulfide near its tip. The extended loop wraps onto the convex surface of the β4-β3-β5 sheet and packs tightly against it. This unusual structural feature is likely important for proper positioning and bracing of the short β2-strand, which forms the primary structural element used by TGFβRII to bind the three TGF-β isoforms.

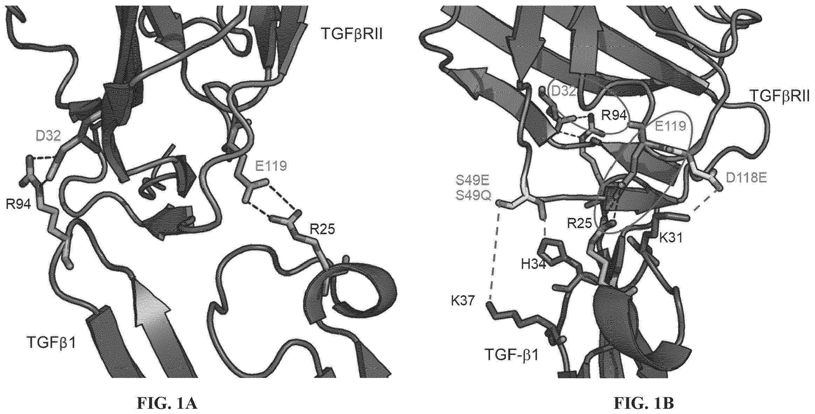

Based on the structure of TGFβ1 in complex with TGFβRII as provided in the present disclosure, the interacting residues are determined. In **FIG. 1A**, TGFβRII amino acid residues (D32 and E119) that form hydrogen bonds with TGFβ1 are highlighted. In **FIG. 1B**, mutated amino acid residues (S49 and D118) that may form additional hydrogen bonds with TGFβ1 are highlighted.

Further analysis shows mutated S49 and D118 in TGFβRII are involved in the interaction with TGFβ1. Thus, in some embodiments, the engineered TGFβRII polypeptide can comprise one or more amino acid mutations at S49 and D118. The position number is determined based on SEQ ID NO: 7.

In some embodiments, the amino acid that corresponds to S49 of SEQ ID NO: 7 is an amino acid with electrically charged side chain, e.g., R, H, K, D, or E. In some embodiments, the amino acid that corresponds to S49 of SEQ ID NO: 7 is an amino acid with a polar uncharged side chain, e.g., T, N, or Q. In some embodiments, the amino acid that corresponds to S49 of SEQ ID NO: 7 has a hydrophobic side chain, e.g., A, V, I, L, M, F, Y, or W. In some embodiments, the amino acid that corresponds to S49 of SEQ ID NO: 7 is C, G, or P. In some embodiments, the amino acid that corresponds to S49 of SEQ ID NO: 7 is not S. In some embodiments, the amino acid that corresponds to S49 of SEQ ID NO: 7 is still S.

In some embodiments, the amino acid that corresponds to S49 forms a hydrogen bond with K37 in TGFβ1. In some embodiments, the amino acid that corresponds to S49 of SEQ ID NO: 7 is E. In some embodiments, the amino acid that corresponds to S49 of SEQ ID NO: 7 is Q.

In some embodiments, the amino acid that corresponds to D118 of SEQ ID NO: 7 is an amino acid with electrically charged side chain, e.g., R, H, K, D, or E. In some embodiments, the amino acid that corresponds to D118 of SEQ ID NO: 7 is an amino acid with a polar uncharged side chain, e.g., T, N, or Q. In some embodiments, the amino acid that corresponds to D118 of SEQ ID NO: 7 has a hydrophobic side chain, e.g., A, V, I, L, M, F, Y, or W. In some embodiments, the amino acid that corresponds to D118 of SEQ ID NO: 7 is C, G, or P. In some embodiments, the amino acid that corresponds to D118 of SEQ ID NO: 7 is not D. In some embodiments, the amino acid that corresponds to D118 of SEQ ID NO: 7 is still D.

In some embodiments, the amino acid that corresponds to D118 of SEQ ID NO: 7 forms a hydrogen bond with K31 in TGFβ1 (based on SEQ ID NO: 15). In some embodiments, the amino acid that corresponds to D118 of SEQ ID NO: 7 is E.

In some embodiments, the amino acid that corresponds to S49 of SEQ ID NO: 7 is E or Q. In some embodiments, the amino acid that corresponds to D118 of SEQ ID NO: 7 is E.

In some embodiments, the engineered TGFβRII polypeptide can have one or more of the following mutations: (a) the amino acid that corresponds to S49 of SEQ ID NO: 7 is E or Q; (b) the amino acid that corresponds to D118 of SEQ ID NO: 7 is E. In some embodiments, the engineered TGFβRII polypeptide has 1, 2, 3, 4, 5, 6, 7, 8, 9, 10 or more than 10 additional mutations, wherein the engineered TGFβRII polypeptide has similar or improved binding affinities with TGFβ1 and/or TGFβ2.

In addition, the analysis in the present disclosure shows that D32 (interacts with R94 of TGFβ1) and E119 (interacts with R25 of TGFβ1) of TGFβRII are the key residues to differentiate the binding affinity of TGFβ1 and TGFβ3 from TGFβ2. Thus, in some embodiments, these amino acid residues are retained. In some embodiments, there amino acid residues are mutated.

Thus, in one aspect, the engineered TGFβRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to any one of SEQ ID NOs: 7-12.

In some embodiments, the engineered TGFβRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to SEQ ID NO: 7.

In some embodiments, the engineered TGFβRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to SEQ ID NO: 8.

In some embodiments, the engineered TGFβRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to SEQ ID NO: 9.

In some embodiments, the engineered TGF-

βRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to SEQ ID NO: 10.

In some embodiments, the engineered TGFβRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to SEQ ID NO: 11.

In some embodiments, the engineered TGFβRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to SEQ ID NO: 12.

In some embodiments, the engineered TGFβRII variants can have at least or about 1 (e.g., at least or about 2, 3, 4, 5, 6, 7, 8, 9, 10, 20, 30, 40) amino acid insertions, deletions, or substitutions as compared to any one of SEQ ID NOs: 7-12.

In some embodiments, the engineered TGFβRII polypeptide has one or more mutations at S49 and D118. In some embodiments, the engineered TGFβRII polypeptide has a mutation at S49. In some embodiments, the engineered TGFβRII polypeptide has a mutation at D118. The position number is determined by SEQ ID NO: 7.

In some embodiments, the engineered TGFβRII polypeptide has one or more of the following mutations:

(a) the amino acid that corresponds to S49 of SEQ ID NO: 7 is not S.
(b) the amino acid that corresponds to D118 of SEQ ID NO: 7 is not D.

In some embodiments, the engineered TGFβRII polypeptide has one or more of the following mutations:

(a) the amino acid that corresponds to S49 of SEQ ID NO: 7 is E or Q;
(b) the amino acid that corresponds to D118 of SEQ ID NO: 7 is E.

In some embodiments, the engineered TGFβRII polypeptide comprises or consists of an amino acid sequence that is at least 80%, 85%, 90%, or 95% identical to any one of SEQ ID NOs: 7-12 with 1, 2, 3, 4, 5, 6, 7, 8, 9, 10, 11, 12, 13, 14, or 15 mutations.

In some embodiments, the engineered TGFβRII polypeptide comprises or consists of about or at least 1, 2, 3, 4, 5, 6, 7, 8, 9, 10, 20, 30, 40, 50, 60, 70, 80, 90, 100, 110, 120, 130, or 140 amino acids. In some embodiments, the engineered TGFβRII polypeptide comprises or consists of no more than 50, 60, 70, 80, 90, 100, 110, 120, 130, 140, 150, or 160 amino acids. In some embodiments, the engineered TGFβRII polypeptide comprises or consists of about or at least 1, 2, 3, 4, 5, 6, 7, 8, 9, 10, 20, 30, 40, 50, 60, 70, 80, 90, 100, 110, 120, 130, or 140 contiguous amino acids from the extracellular domain of the engineered TGFβRII polypeptide. In some embodiments, the engineered TGFβRII variants can have at least or about 1 (e.g., at least or about 2, 3, 4, 5, 6, 7, 8, 9, 10, 20, 30, 40) amino acid insertions, deletions, or substitutions.

In some embodiments, the TGFβ binding domain in the engineered TGFβRII polypeptide comprises or consists of about or at least 1, 2, 3, 4, 5, 6, 7, 8, 9, 10, 20, 30, 40, 50, 60, 70, 80, 90, 100, 110, 120, 130, or 140 amino acids. In some embodiments, the TGFβ binding domain in the engineered TGFβRII polypeptide comprises or consists of no more than 50, 60, 70, 80, 90, 100, 110, 120, 130, 140, 150, or 160 amino acids. In some embodiments, the TGFβ binding domain in the engineered TGFβRII polypeptide comprises or consists of about or at least 1, 2, 3, 4, 5, 6, 7, 8, 9, 10, 20, 30, 40, 50, 60, 70, 80, 90, 100, 110, 120, 130, or 140 contiguous amino acids from the extracellular domain of the engineered TGFβRII polypeptide. In some embodiments, the TGFβ binding domain in the engineered TGFβRII variants can have at least or about 1 (e.g., at least or about 2, 3, 4, 5, 6, 7, 8, 9, 10, 20, 30, 40) amino acid insertions, deletions, or substitutions.

The engineered TGFβRII polypeptide can have additional modifications. In some embodiments, the engineered TGFβRII polypeptide (or the TGFβ binding domain) can have a CH2 domain and/or a CH3 domain of Fc. In some embodiments, the engineered TGFβRII polypeptide (or the TGFβ binding domain) can be linked to the N-terminal of the CH2 domain (e.g., through an optional hinge region or a GS linker). In some embodiments, the engineered TGFβRII polypeptide (or the TGFβ binding domain) can be linked to the C-terminal of the CH3 domain (e.g., through an optional GS linker). In some embodiments, the hinge region is an IgG hinge region (e.g., IgG1, IgG2, IgG3, IgG4 hinge region). In some embodiments, the CH2 domain is an IgG CH2 domain (e.g., IgG1, IgG2, IgG3, IgG4 CH2 domain). In some embodiments, the CH3 domain is an IgG CH3 domain (e.g., IgG1, IgG2, IgG3, IgG4 CH3 domain).

In some embodiments, the engineered TGFβRII polypeptide comprises or consists of an amino acid sequence that is at least 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% identical to any one of SEQ ID NOs: 1-12.

**TGFβRII protein constructs**

The disclosure also provides engineered TGFβRII protein constructs that can specifically bind to TGFβ (e.g., TGFβ1 or TGFβ3). In some embodiments, these protein constructs do not bind to TGFβ2. In some embodiments, these protein constructs can block TGFβRII signaling pathway. In some embodiments, these protein constructs can improve the tumor microenvironment.

In some embodiments, the engineered TGF-βRII polypeptide is linked to Fc. In some embodiments, the engineered TGFβRII polypeptide is linked to the N-terminus of the Fc (e.g., through a hinge region) or the C-terminus of the Fc (e.g., through a linker sequence).

In some embodiments, the engineered TGF-βRII protein construct comprises any engineered TGF-βRII variant as described herein. In some embodiments, the engineered TGFβRII protein construct has a sequence that is at least 80%, 81%, 82%, 83%, 84%, 85%, 86%, 87%, 88%, 89% 90%, 91%, 92%, 93%, 94%, 95%, 96%, 97%, 98%, 99% or 100% identical to any one of SEQ ID NOs: 1-6.

The disclosure also provides nucleic acid comprising a polynucleotide encoding a polypeptide comprising a sequence that is at least 80%, 81%, 82%, 83%, 84%, 85%, 86%, 87%, 88%, 89% 90%, 91%, 92%, 93%, 94%, 95%, 96%, 97%, 98%, 99% or 100% identical to any amino sequence as described herein, e.g., SEQ ID NOs: 1-14.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences. For example, the comparison of sequences and determination of percent identity between two sequences can be accomplished using a Blossum 62 scoring matrix with a gap penalty of 12, a gap extend penalty of 4, and a frameshift gap penalty of 5. In addition, the term "corresponding to" is often used to designate the position/identity of a residue in a polymer, such as an amino acid residue in a polypeptide or a nucleotide residue in a nucleic acid. Those of ordinary skill will appreciate that, for purposes of simplicity, residues in such a polymer are often designated using a numbering system based on a reference polymer, so that a residue in a first sequence "corresponding to" a residue at position 49 in the reference polymer, for example, need not actually be the 49th residue in the first polymer but rather corresponds to the residue found at the 49th position in the reference polymer based on alignment. Those of ordinary skill in the art readily appreciate how to identify "corresponding" amino acids, including through use of one or more commercially-available algorithms specifically designed for polymer sequence comparisons.

The engineered TGFβRII protein construct further comprises an Fc region of an antibody. These antibodies can be of any type (e.g., IgG, IgE, IgM, IgD, IgA, and IgY), class or subclass (e.g., IgG1, IgG2, IgG3, IgG4, IgA1, IgA2, IgE1, IgE2). In some embodiments, the he Fc region is derived from human IgG (e.g., IgG1, IgG2, IgG3, or IgG4). In some embodiments, the Fc region is an IgG4 Fc region (e.g., human IgG4 Fc region).

In some embodiments, the engineered TGF-βRII variant is linked to the Fc region through an antibody hinge region (e.g., IgG, IgE hinge region). In addition, the Fc region can be modified to provide desired effector functions or serum half-life.

In some embodiments, the engineered TGF-βRII variant is linked to the Fc through a linker sequence (e.g., SEQ ID NO: 14).

In some embodiments, the engineered TGF-βRII protein construct further comprises an antibody (e.g., bispecific antibody). In some embodiments, the engineered TGFβRII variant is linked to the C-terminus of the antibody (e.g., bispecific antibody). In some embodiments, the engineered TGFβRII variant is linked to the C-terminus of the antibody (e.g., bispecific antibody) via a peptide linker. In some embodiments, the engineered TGFβRII variant is linked to the N-terminus of the antibody (e.g., bispecific antibody). In some embodiments, the engineered TGFβRII variant is linked to an antigen-binding domain of the antibody.

## Characteristics of the engineered TGFβRII variants and protein constructs

The engineered TGFβRII variants and protein constructs described herein can block the binding between TGFβ (e.g., TGFβ1 and/or TGFβ2) and endogenous TGFβRII that are expressed on the surface of tumor cells, fibroblasts, or immune cells. In some embodiments, by binding to TGFβ, the engineered TGFβRII variants and protein constructs can inhibit the binding of TGFβ to endogenous TGFβRII, thereby blocking TGFB/TGFβRII pathway.

In some implementations, the engineered TGFβRII variants and protein constructs can bind to TGFβ (e.g., human TGFβ1, human TGFβ3) with a dissociation rate ($k_{off}$) of less than 0.1 $s^{-1}$, less than 0.01 $s^{-1}$, less than 0.001 $s^{-1}$, less than 0.0001 $s^{-1}$, or less than 0.00001 $s^{-1}$. In some embodiments, the dissociation rate ($k_{off}$) is greater than 0.01 $s^{-1}$, greater than 0.001 $s^{-1}$, greater than 0.0001 $s^{-1}$, greater than 0.00001 $s^{-1}$, or greater than 0.000001 $s^{-1}$.

In some embodiments, kinetic association rates ($k_{on}$) is greater than $1 \times 10^2$/Ms, greater than $1 \times 10^3$/Ms, greater than $1 \times 10^4$/Ms, greater than $1 \times 10^5$/Ms, or greater than $1 \times 10^6$/Ms. In some embodiments, kinetic association rates ($k_{on}$) is less than $1 \times 10^5$/Ms, less than

$1 \times 10^6$/Ms, or less than $1 \times 10^7$/Ms.

Affinities can be deduced from the quotient of the kinetic rate constants ($KD=k_{off}/k_{on}$). In some embodiments, KD is less than $1 \times 10^{-6}$ M, less than $1 \times 10^{-7}$ M, less than $1 \times 10^{-8}$ M, less than $1 \times 10^{-9}$ M, or less than $1 \times 10^{-10}$ M. In some embodiments, the KD is less than 300 nM, 200 nM, 100 nM, 50nM, 30 nM, 20 nM, 15 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, or 10 pM. In some embodiments, KD is greater than $1 \times 10^{-7}$M, greater than $1 \times 10^{-8}$ M, greater than $1 \times 10^{-9}$ M, greater than $1 \times 10^{-10}$ M, greater than $1 \times 10^{-11}$ M, or greater than $1 \times 10^{-12}$ M.

General techniques for measuring the affinity include, e.g., ELISA, RIA, surface plasmon resonance (SPR), and Bio-Layer Interferometry (BLI). In some embodiments, the engineered TGFβRII variants and protein constructs can bind to monkey TGFβ (e.g., TGFβ1, TGFβ2, TGFβ3), and/or mouse TGFβ (e.g., TGFβ1, TGFβ2, TGFβ3). In some embodiments, the engineered TGFβRII variants and protein constructs cannot bind to monkey TGFβ (e.g., TGFβ1, TGFβ2, TGFβ3), and/or mouse TGFβ (e.g., TGFβ1, TGFβ2, TGFβ3).

In some embodiments, the engineered TGFβRII variants cannot bind to TGFβ2 (e.g., human TGFβ2).

In some embodiments, thermal stabilities are determined. The engineered TGFβRII variants and protein constructs as described herein can have a Tm greater than 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, or 95 °C. In some embodiments, Tm is less than 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, or 95 °C.

In some embodiments, the engineered TGFβRII variants and/or protein constructs thereof interacts with R94, R25, K37, and/or K31 on TGFβ1.

In some embodiments, the engineered TGFβRII variants and/or protein constructs thereof interacts with R94, R25, K37, and/or K31 on TGFβ3.

In some embodiments, the engineered TGFβRII variants and/or protein constructs thereof interacts with R94, R25, K37, and/or K31 on TGFβ1 via hydrogen bond interactions.

In some embodiments, the engineered TGFβRII variants and/or protein constructs thereof interacts with R94, R25, K37, and/or K31 on TGFβ3 via hydrogen bond interactions.

In some embodiments, the engineered TGFβRII variants and/or protein constructs thereof as described herein can capture TGFβ (e.g., TGFβ1 or TGFβ3) and thereby improve tumor microenvironment.

In some embodiments, the engineered TGFβRII variants and/or protein constructs thereof as described herein can inhibit tumor growth.

In some embodiments, the engineered TGF-βRII variants and/or protein constructs as described herein has a tumor growth inhibition percentage (TGI%) that is greater than 10%, 20%, 30%, 40%, 50%, 60%, 70%, 80%, 90%, 100%, 110%, 120%, 130%, 140%, 150%, 160%, 170%, 180%, 190%, or 200%. In some embodiments, the engineered TGFβRII variants and/or protein constructs as described herein has a tumor growth inhibition percentage that is less than 60%, 70%, 80%, 90%, 100%, 110%, 120%, 130%, 140%, 150%, 160%, 170%, 180%, 190%, or 200%. The TGI% can be determined, e.g., at 3, 4, 5, 6, 7, 8, 9, 10, 11, 12, 13, 14, 15, 16, 17, 18, 19, 20, 21, 22, 23, 24, 25, 26, 27, 28, 29, or 30 days after the treatment starts, or 1, 2, 3, 4, 5, 6, 7, 8, 9, 10, 11, or 12 months after the treatment starts. As used herein, the tumor growth inhibition percentage (TGI%) is calculated using the following formula:

$$TGI\,(\%) = [1 - (Ti - T0)/(Vi - V0)] \times 100$$

Ti is the average tumor volume in the treatment group on day i. T0 is the average tumor volume in the treatment group on day zero. Vi is the average tumor volume in the control group on day i. V0 is the average tumor volume in the control group on day zero.

In some embodiments, the protein constructs as described herein have a functional Fc region. In some embodiments, the Fc region is human IgG1, human IgG2, human IgG3, or human IgG4. In some embodiments, effector function of a functional Fc region is antibody-dependent cell-mediated cytotoxicity (ADCC). In some embodiments, effector function of a functional Fc region is phagocytosis. In some embodiments, effector function of a functional Fc region is ADCC and phagocytosis. In some embodiments, the protein constructs as described herein have an Fc region without effector function. In some embodiments, the Fc is a human IgG4 Fc. In some embodiments, the Fc does not have a functional Fc region. For example, the Fc region has LALA mutations (L234A and L235A mutations in EU numbering), or LALA-PG mutations (L234A, L235A, P329G mutations in EU numbering).

Some other modifications to the Fc region can be made. For example, a cysteine residue(s) can be introduced into the Fc region, thereby allowing interchain disulfide bond formation in this region. The homodimeric fusion protein thus generated may have any increased half-life *in vitro* and/or *in vivo*.

In some embodiments, the IgG4 has S228P mutation (EU numbering). The S228P mutation prevents in vivo and in vitro IgG4 Fab-arm exchange.

In some embodiments, Fc regions are provided having a carbohydrate structure that lacks fucose attached (directly or indirectly) to an Fc region. For example, the amount of fucose in such Fc region composition may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e.g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues; or position 314 in Kabat numbering); however, Asn297 may also be located about ±3 amino acids upstream or downstream of position 297, i.e., between positions 294 and 300, due to minor sequence variations in Fc region sequences. Such fucosylation variants may have improved ADCC function. In some embodiments, to reduce glycan heterogeneity, the Fc region can be further engineered to replace the Asparagine at position 297 with Alanine (N297A).

In some embodiments, the binding affinity between TGFβ (e.g., TGFβ1 and/or TGFβ3) and the engineered TGFβRII variants and/or protein constructs as described herein is at least 1-fold, 2-fold, 3-fold, 4-fold, 5-fold, 6-fold, 7-fold, 8-fold, 9-fold, 10-fold, 15-fold, 20-fold, 25-fold, 30-fold, 35-fold, 40-fold, 45-fold, or 50-fold stronger as compared to that between TGFβ and a wild-type TGFβRII or protein constructs thereof.

In some embodiments, the main peak of HPLC-SEC accounts for at least 30%, 40%, 50%, 60%, 70%, 80%, 90%, 95%, 96%, 97%, 98%, 99%, or 99.5% of the engineered TGFβRII variants and/or protein constructs described herein after purification by a protein A column.

In some embodiments, the engineered TGF-βRII variants and/or protein constructs as described herein has an expression yield that is higher than 50 mg/L, higher than 100 mg/L, higher than 200 mg/L, higher than 300 mg/L, or higher than 400 mg/L.

In some embodiments, the engineered TGF-βRII variants and/or protein constructs thereof as described herein can block the interaction between human TGFβ and human TGFβRII. In some embodiments, the engineered TGFβRII variants and/or protein constructs thereof as described herein can inhibit TGFβ-induced signaling in HEK293T cells. In some embodiments, the blocking ability of the engineered TGFβRII variants and/or protein constructs thereof as described herein is at least at least 50%, at least 60%, at least 70%, at least 80%, at least 90%, at least 100%, at least 110%, at least 120%, at least 130%, at least 140%, or at least 150% as compared to that an anti-TGFβ reference antibody. In some embodiments, the blocking ability of the engineered TGFβRII variants and/or protein constructs thereof as described herein is at least 1-fold, 2-fold, 3-fold, 4-fold, 5-fold, 6-fold, 7-fold, 8-fold, 9-fold, 10-fold, 15-fold, 20-fold, 25-fold, 30-fold, 35-fold, 40-fold, 45-fold, or 50-fold stronger as compared to that a wild-type TGFβRII or protein constructs thereof. In some embodiments, the IC50 value of engineered TGFβRII variants and/or protein constructs thereof to block the TGFβ/TGFβRII interaction is less than 2 nM, less than 1 nM, less than 0.5 nM, less than 0.4 nM, less than 0.3 nM, less than 0.2 nM, or less than 0.1 nM.

## Methods of making engineered TGFβRII variants and protein constructs

Variants of the TGFβRII described herein can be prepared by introducing appropriate nucleotide changes into the DNA encoding a TGFβRII peptide or a part thereof or by peptide synthesis. Such variants include, for example, deletions, insertions, or substitutions of residues within the amino acids sequences.

Screening can be performed. In a population of such variants, some engineered TGFβRII variants will have increased affinity for the TGFβ. Any combination of deletions, insertions, and/or combinations can be made to arrive at a variant that has increased binding affinity for the target. The amino acid changes introduced into the variant can also alter or introduce new post-translational modifications into the polypeptide, such as changing (e.g., increasing or decreasing) the number of glycosylation sites, changing the type of glycosylation site (e.g., changing the amino acid sequence such that a different sugar is attached by enzymes present in a cell), or introducing new glycosylation sites.

Engineered TGFβRII variants can be derived from any species of animal, including mammals. Non-limiting examples of TGFβRII variants include TGFβRII variants derived from humans, primates, e.g., monkeys and apes, cows, pigs, horses, sheep, camelids (e.g., camels and llamas), chicken, goats, and rodents (e.g., rats, mice, hamsters and rabbits).

The present disclosure also provides recombinant vectors (e.g., an expression vectors) that include an isolated polynucleotide disclosed herein (e.g., a polynucleotide that encodes a polypeptide disclosed herein), host cells into which are introduced the recombinant vectors (i.e., such that the host cells contain the polynucleotide and/or a vector comprising the polynucleotide), and the production of recombinant polypeptides or fragments thereof by recombinant techniques.

As used herein, a "vector" is any construct capable of delivering one or more polynucleotide(s) of interest to a host cell when the vector is introduced to the host cell. An "expression vector" is capable of delivering and expressing the one or more polynucleotide(s) of interest as an encoded polypeptide in a host cell into which the expression vector has been introduced. Thus, in an expression vector, the polynucleotide of interest is positioned for expression in the vector by being operably linked with regulatory elements such as a promoter, enhancer, and/or a poly-A tail, either within the vector or in the genome of the host cell at or near or flanking the integration site of the polynucleotide of interest such that the polynucleotide of interest will be translated in the host cell introduced with the expression vector.

A vector can be introduced into the host cell by methods known in the art, e.g., electroporation, chemical transfection (e.g., DEAE-dextran), transformation, transfection, and infection and/or transduction (e.g., with recombinant virus). Thus, non-limiting examples of vectors include viral vectors (which can be used to generate recombinant virus), naked DNA or RNA, plasmids, cosmids, phage vectors, and DNA or RNA expression vectors associated with cationic condensing agents.

In some implementations, a polynucleotide disclosed herein (e.g., a polynucleotide that encodes a polypeptide disclosed herein) is introduced using a viral expression system (e.g., vaccinia or other pox virus, retrovirus, or adenovirus), which may involve the use of a non-pathogenic (defective), replication competent virus, or may use a replication defective virus. Techniques for incorporating DNA into such expression systems are well known to those of ordinary skill in the art. The DNA may also be "naked." The uptake of naked DNA may be increased by coating the DNA onto biodegradable beads that are efficiently transported into the cells.

For expression, the DNA insert comprising a polypeptide-encoding polynucleotide disclosed herein can be operatively linked to an appropriate promoter (e.g., a heterologous promoter), such as the phage lambda PL promoter, the *E. coli* lac, trp and tac promoters, the SV40 early and late promoters and promoters of retroviral LTRs, to name a few. Other suitable promoters are known to the skilled artisan. In some embodiments, the promoter is a cytomegalovirus (CMV) promoter. The expression constructs can further contain sites for transcription initiation, termination and, in the transcribed region, a ribosome binding site for translation. The coding portion of the mature transcripts expressed by the constructs may include a translation initiating at the beginning and a termination codon (UAA, UGA, or UAG) appropriately positioned at the end of the polypeptide to be translated.

As indicated, the expression vectors can include at least one selectable marker. Such markers include dihydrofolate reductase or neomycin resistance for eukaryotic cell culture and tetracycline or ampicillin resistance genes for culturing in *E. coli* and other bacteria. Representative examples of appropriate hosts include, but are not limited to, bacterial cells, such as *E. coli, Streptomyces,* and *Salmonella typhimurium* cells; fungal cells, such as yeast cells; insect cells such as Drosophila S2 and Spodoptera Sf9 cells; animal cells such as CHO, COS, Bowes melanoma, and HK 293 cells; and plant cells. Appropriate culture mediums and conditions for the host cells described herein are known in the art.

Non-limiting vectors for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Non-limiting eukaryotic vectors include pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Non-limiting bacterial promoters suitable for use include the *E. coli* lacI and lacZ promoters, the T3 and T7 promoters, the gpt promoter, the lambda PR and PL promoters and the trp promoter. Suitable eukaryotic promoters include the CMV immediate early promoter, the HSV thymidine kinase promoter, the early and late SV40 promoters, the promoters of retroviral LTRs, such as those of the Rous sarcoma virus (RSV), and metallothionein promoters, such as the mouse metallothionein-I promoter.

In the yeast *Saccharomyces cerevisiae,* a number of vectors containing constitutive or inducible promoters such as alpha factor, alcohol oxidase, and PGH can be used.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al., Basic Methods In Molecular Biology (1986), which is incorporated herein by reference in its entirety.

Transcription of DNA encoding a polypeptide of the present disclosure by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at base pairs 100 to 270, the cytomegalovirus early promoter enhancer, the polyoma enhancer on the late side of the replication origin, and adenovirus enhancers.

For secretion of the translated protein into the lumen of the endoplasmic reticulum, into the periplasmic space or into the extracellular environment, appropriate secretion signals may be incorporated into the expressed polypeptide. The signals may be endogenous to the polypeptide or they may be heterologous signals.

The polypeptide (e.g., TGFβRII variants) can be expressed in a modified form, such as a fusion protein (*e.g.*, a GST-fusion) or with a histidine-tag, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties can be added to the polypeptide to facilitate purification. Such regions can be removed prior to final preparation of the polypeptide. The addition of peptide moieties to polypeptides to engender secretion or excretion, to improve stability and to facilitate purification, among others, are familiar and routine techniques in the art.

## Methods of Treatment

The engineered TGFβRII variants and protein constructs of the present disclosure can be used for various therapeutic purposes.

In one aspect, the disclosure provides methods for treating a cancer in a subject, methods of reducing the rate of the increase of volume of a tumor in a subject over time, methods of reducing the risk of developing a metastasis, or methods of reducing the risk of developing an additional metastasis in a subject. In some embodiments, the treatment can halt, slow, retard, or inhibit progression of a cancer. In some embodiments, the treatment can result in the reduction of in the number, severity, and/or duration of one or more symptoms of the cancer in a subject.

In one aspect, the disclosure features methods that include administering a therapeutically effective amount of engineered TGFβRII variants and protein constructs disclosed herein to a subject in need thereof (e.g., a subject having, or identified or diagnosed as having, a cancer), e.g., breast cancer (e.g., triple-negative breast cancer), carcinoid cancer, cervical cancer, endometrial cancer, glioma, head and neck cancer, liver cancer, lung cancer, small cell lung cancer, lymphoma, melanoma, ovarian cancer, pancreatic cancer, prostate cancer, renal cancer, colorectal cancer, gastric cancer, testicular cancer, thyroid cancer, bladder cancer, urethral cancer, or hematologic malignancy. In some embodiments, the cancer is unresectable melanoma or metastatic melanoma, non-small cell lung carcinoma (NSCLC), small cell lung cancer (SCLC), bladder cancer, or metastatic hormone-refractory prostate cancer. In some embodiments, the subject has a solid tumor. In some embodiments, the cancer is squamous cell carcinoma of the head and neck (SCCHN), renal cell carcinoma (RCC), triple-negative breast cancer (TNBC), or colorectal carcinoma. In some embodiments, the cancer is melanoma, pancreatic carcinoma, mesothelioma, hematological malignancies, especially Non-Hodgkin's lymphoma, lymphoma, chronic lymphocytic leukemia, or advanced solid tumors. In some embodiments, the cancer is breast cancer, ovarian cancer, glioma, colon cancer, hepatocellular carcinoma, prostate cancer, renal cell carcinoma, melanoma, lung cancer, pancreatic cancer, or hepatoma.

In some embodiments, the compositions and methods disclosed herein can be used for treatment of patients at risk for a cancer. Patients with cancer can be identified with various methods known in the art.

In some embodiments, the methods described herein can be used to treat serious blood disorders, e.g., anemia.

In some embodiments, the method described herein or the engineered TGFβRII variants and protein constructs of the present disclosure can be used to treat cancer, cystic fibrosis, and/or anemia caused by ineffective erythropoiesis (e.g., myelodysplastic syndromes associated anemia, acquired aplastic anemia, or Fanconi anemia).

As used herein, by an "effective amount" is meant an amount or dosage sufficient to effect beneficial or desired results. In some embodiments, the desired results can include halting, slowing, retarding, or inhibiting progression of a disease, e.g., a cancer. An effective amount will vary depending upon, e.g., an age and a body weight of a subject to which the engineered TGF-βRII variants and protein constructs, vector comprising the polynucleotide encoding the engineered TGFβRII variants and protein constructs, and/or compositions thereof is to be administered, a severity of symptoms and a route of administration, and thus administration can be determined on an individual basis.

An effective amount can be administered in one or more administrations. By way of example, an effective amount of the engineered TGFβRII variants and/or protein constructs is an amount sufficient to ameliorate, stop, stabilize, reverse, inhibit, slow and/or delay progression of a cancer in a patient or is an amount sufficient to ameliorate, stop, stabilize, reverse, slow and/or delay proliferation of a cell (e.g., a biopsied cell, any of the cancer cells described herein, or cell line (e.g., a cancer cell line)) *in vitro*. As is understood in the art, an effective amount may vary, depending on, *inter alia,* patient history as well as other factors such as the type (and/or dosage) of the engineered TGFβRII variants and protein constructs used.

Effective amounts and schedules for administering the engineered TGFβRII variants and protein constructs, the polynucleotides encoding the engineered TGFβRII variants and protein constructs, and/or compositions disclosed herein may be determined empirically, and making such determinations is within the skill in the art. Those skilled in the art will understand that the dosage that must be administered will vary depending on, for example, the mammal that will receive the engineered TGFβRII variants and protein constructs, the polynucleotides, and/or compositions disclosed herein, the route of administration, the particular type of polynucleotides, and/or compositions disclosed herein used and other drugs being administered to the mammal.

A typical daily dosage of an effective amount of the engineered TGFβRII variants and/or protein constructs is 0.1 mg/kg to 100 mg/kg (mg per kg of patient weight). In some embodiments, the dosage can be less than 100 mg/kg, 10 mg/kg, 9 mg/kg, 8 mg/kg, 7 mg/kg, 6 mg/kg, 5 mg/kg, 4 mg/kg, 3 mg/kg, 2 mg/kg, 1 mg/kg, 0.5 mg/kg, or 0.1 mg/kg. In some embodiments, the dosage can be greater than 10 mg/kg, 9 mg/kg, 8 mg/kg, 7 mg/kg, 6 mg/kg, 5 mg/kg, 4 mg/kg, 3 mg/kg, 2 mg/kg, 1 mg/kg, 0.5 mg/kg, or 0.1 mg/kg. In some embodiments, the dosage is about 10 mg/kg, 9 mg/kg, 8 mg/kg, 7 mg/kg, 6 mg/kg, 5 mg/kg, 4 mg/kg, 3 mg/kg, 2 mg/kg, or 1 mg/kg. In some embodiments, the dosage is about 1 to 10 mg/kg, about 1 to 5 mg/kg, or about 2 to 5 mg/kg.

In any of the methods described herein, the engineered TGFβRII variants and protein constructs can be administered to the subject at least once a week (e.g., once a week, twice a week, three times a week, four times a week, once a day, twice a day, or three times a day).

In some embodiments, the one or more additional therapeutic agents can be administered to the subject prior to, or after administering the engineered TGFβRII variants and protein constructs. In some embodiments, the one or more additional therapeutic agents are administered to the subject such that there is an overlap in the bioactive period of the one or more additional therapeutic agents and the engineered TGFβRII variants and protein constructs in the subject.

In some embodiments, one or more additional therapeutic agents can be administered to the subject. The additional therapeutic agent can comprise one or more inhibitors selected from the group consisting of an inhibitor of B-Raf, an EGFR inhibitor, an inhibitor of a MEK, an inhibitor of ERK, an inhibitor of K-Ras, an inhibitor of c-Met, an inhibitor of anaplastic lymphoma kinase (ALK), an inhibitor of a phosphatidylinositol 3-kinase (PI3K), an inhibitor of an Akt, an inhibitor of mTOR, a dual PI3K/mTOR inhibitor, an inhibitor of Bruton's tyrosine kinase (BTK), and an inhibitor of Isocitrate dehydrogenase 1 (IDH1) and/or Isocitrate dehydrogenase 2 (IDH2). In some embodiments, the additional therapeutic agent is an inhibitor of indoleamine 2,3-dioxygenase-1) (IDO1) (e.g., epacadostat).

In some embodiments, the additional therapeutic agent can comprise one or more inhibitors selected from the group consisting of an inhibitor of HER3, an inhibitor of LSD1, an inhibitor of MDM2, an inhibitor of BCL2, an inhibitor of CHK1, an inhibitor of activated hedgehog signaling pathway, and an agent that selectively degrades the estrogen receptor.

In some embodiments, the additional therapeutic agent can comprise one or more therapeutic agents selected from the group consisting of Trabectedin, nab-paclitaxel, Trebananib, Pazopanib, Cediranib, Palbociclib, everolimus, fluoropyrimidine, IFL, regorafenib, Reolysin, Alimta, Zykadia, Sutent, temsirolimus, axitinib, everolimus, sorafenib, Votrient, Pazopanib, IMA-901, AGS-003, cabozantinib, Vinflunine, an Hsp90 inhibitor, Ad-GM-CSF, Temazolomide, IL-2, IFNa, vinblastine, Thalomid, dacarbazine, cyclophosphamide, lenalidomide, azacytidine, lenalidomide, bortezomid, amrubicine, carfilzomib, pralatrexate, and enzastaurin.

In some embodiments, the additional therapeutic agent can comprise one or more therapeutic agents selected from the group consisting of an adjuvant, a TLR agonist, tumor necrosis factor (TNF) alpha, IL-1, HMGB1, an IL-10 antagonist, an IL-4 antagonist, an IL-13 antagonist, an IL-17 antagonist, an HVEM antagonist, an ICOS agonist, a treatment targeting CX3CL1, a treatment targeting CXCL9, a treatment targeting CXCL10, a treatment targeting CCL5, an LFA-1 agonist, an ICAM1 agonist, and a Selectin agonist.

In some embodiments, carboplatin, nab-paclitaxel, paclitaxel, cisplatin, pemetrexed, gemcitabine, FOLFOX, or FOLFIRI are administered to the subject.

In some embodiments, the additional therapeutic agent is an anti-OX40 antibody, an anti-PD-1 antibody, an anti-PD-L1 antibody, an anti-PD-L2 antibody, an anti-TGFβRII antibody, an anti-TGFβ antibody, an anti-LAG-3 antibody, an anti-TIGIT antibody, an anti-BTLA antibody, an anti-CTLA-4 antibody, or an anti-GITR antibody. In some embodiments, the additional therapeutic agent is an anti-CD20 antibody (e.g., rituximab) or an anti-EGF receptor antibody (e.g., cetuximab).

## Pharmaceutical Compositions and Routes of Administration

Also provided herein are pharmaceutical compositions that contain the engineered TGFβRII variants and protein constructs described herein. The pharmaceutical compositions can be formulated in any manner known in the art.

Pharmaceutical compositions are formulated to be compatible with their intended route of administration (e.g., intravenous, intraarterial, intramuscular, intradermal, subcutaneous, or intraperitoneal). The compositions can include a sterile diluent (e.g., sterile water or saline), a fixed oil, polyethylene glycol, glycerine, propylene glycol or other synthetic solvents, antibacterial or antifungal agents, such as benzyl alcohol or methyl parabens, chlorobutanol, phenol, ascorbic acid, thimerosal, and the like, antioxidants, such as ascorbic acid or sodium bisulfite, chelating agents, such as ethylenediaminetetraacetic acid, buffers, such as acetates, citrates, or phosphates, and isotonic agents, such as sugars (e.g., dextrose), polyalcohols (e.g., mannitol or sorbitol), or salts (e.g., sodium chloride), or any combination thereof. Liposomal suspensions can also be used as pharmaceutically acceptable carriers. Preparations of the compositions can be formulated and enclosed in ampules, disposable syringes, or multiple dose vials. Where required (as in, for example, injectable formulations), proper fluidity can be maintained by, for example, the use of a coating, such as lecithin, or a surfactant. Absorption of the agents can be prolonged by including an agent that delays absorption (e.g., aluminum monostearate and gelatin). Alternatively, controlled release can be achieved by implants and microencapsulated delivery systems, which can include biodegradable, biocompatible polymers (e.g., ethylene vinyl acetate, polyanhydrides, polyglycolic acid, collagen, polyorthoesters, and polylactic acid).

Compositions containing the engineered TGFβRII variants and protein constructs described herein can be formulated for parenteral (e.g., intravenous, intraarterial, intramuscular, intradermal, subcutaneous, or intraperitoneal) administration in dosage unit form (i.e., physically discrete units containing a predetermined quantity of active compound for ease of administration and uniformity of dosage).

Pharmaceutical compositions for parenteral administration are preferably sterile and substantially isotonic and manufactured under Good Manufacturing Practice (GMP) conditions. Pharmaceutical compositions can be provided in unit dosage form (i.e., the dosage for a single administration). Pharmaceutical compositions can be formulated using one or more physiologically acceptable carriers, diluents, excipients or auxiliaries. The formulation depends on the route of administration chosen. For injection, the engineered TGFβRII variants and protein constructs can be formulated in aqueous solutions, preferably in physiologically-compatible buffers to reduce discomfort at the site of injection. The solution can contain formulatory agents such as suspending, stabilizing and/or dispersing agents. Alternatively the engineered TGFβRII variants and protein constructs can be in lyophilized form for constitution with a suitable vehicle, e.g., sterile pyrogen-free water, before use.

Toxicity and therapeutic efficacy of compositions can be determined by standard pharmaceutical procedures in cell cultures or experimental animals (e.g., monkeys). One can, for example, determine the LD50 (the dose lethal to 50% of the population) and the ED50 (the dose therapeutically effective in 50% of the population): the therapeutic index being the ratio of LD50:ED50. Agents that exhibit high therapeutic indices are preferred. Where an agent exhibits an undesirable side effect, care should be taken to minimize potential damage (i.e., reduce unwanted side effects). Toxicity and therapeutic efficacy can be determined by other standard pharmaceutical procedures.

Exemplary doses include milligram or microgram amounts of any of the engineered TGFβRII variants and protein constructs described herein per kilogram of the subject's weight (e.g., about 1 μg/kg to about 500 mg/kg; about 100 μg/kg to about 500 mg/kg; about 100 μg/kg to about 50 mg/kg; about 10 μg/kg to about 5 mg/kg; about 10 μg/kg to about 0.5 mg/kg; about 1 μg/kg to about 50 μg/kg; about 1 mg/kg to about 10 mg/kg; or about 1 mg/kg to about 5 mg/kg). While these doses cover a broad range, one of ordinary skill in the art will understand that therapeutic agents can vary in their potency, and effective amounts can be determined by methods known in the art. Typically, relatively low doses are administered at first, and the attending health care professional or veterinary professional (in the case of therapeutic application) or a researcher (when still working at the development stage) can subsequently and gradually increase the dose until an appropriate response is obtained. In addition, it is understood that the specific dose level for any particular subject will depend upon a variety of factors including the activity of the specific compound employed, the age, body weight, general health, gender, and diet of the subject, the time of administration, the route of administration, the rate of excretion, and the half-life of the engineered TGFβRII variants and protein constructs *in vivo*.

The pharmaceutical compositions can be included in a container, pack, or dispenser together with instructions for administration. The disclosure also provides methods of manufacturing the engineered TGFβRII variants and protein constructs for various uses as described herein.

## EXAMPLES

The invention is further described in the following examples, which do not limit the scope of the invention described in the claims.

### Example 1. Design of engineered TGFβRII variants

The 3D structure of the interface between the TGFβRII extracellular domain and its ligand TGFβ1 is shown in **FIG. 1A**.

A detailed analysis of TGFβRII/ TGFβ1 complex structure was performed. From the structure, it was determined that D32 (interacts with R94 of TGFβ1) and E119 (interacts with R25 of TGFβ1) of TGFβRII are the key residues to differentiate the binding affinity of TGFβ1 and TGFβ3 from TGFβ2. Thus, the analysis indicates that the engineered TGFβRII variants should preferably retain D32 and E119.

There are two major hydrogen-bond interactions (D32-R94 and E119-R25) between TGFβRII and TGFβ1. To increase the binding ability of TGFβRII to TGFβ1, it is desirable to add additional hydrogen bond interactions.

Based on the finding above, it was determined that mutations at S49 and D118 of TGFβRII can be important to increase the binding affinity of TGFβRII to TGFβ1, as the mutated TGFβRII will have two additional hydrogen-bond interactions (S49E/Q - K37 and D118E-K31).

K31 is conserved among TGFβ1, TGFβ2, and TGFβ3. To determine whether the binding affinity of mutated TGFβRII to TGFβ2 is changed, the binding affinities were examined.

In order to find engineered TGFβRII variants having higher binding affinities to TGFβ1 than wild-type TGFβRII, S49 and/or D118 were mutated. The amino acid position is based on the sequence of wildtype TGFβRII extracellular domain (SEQ ID NO: 7).

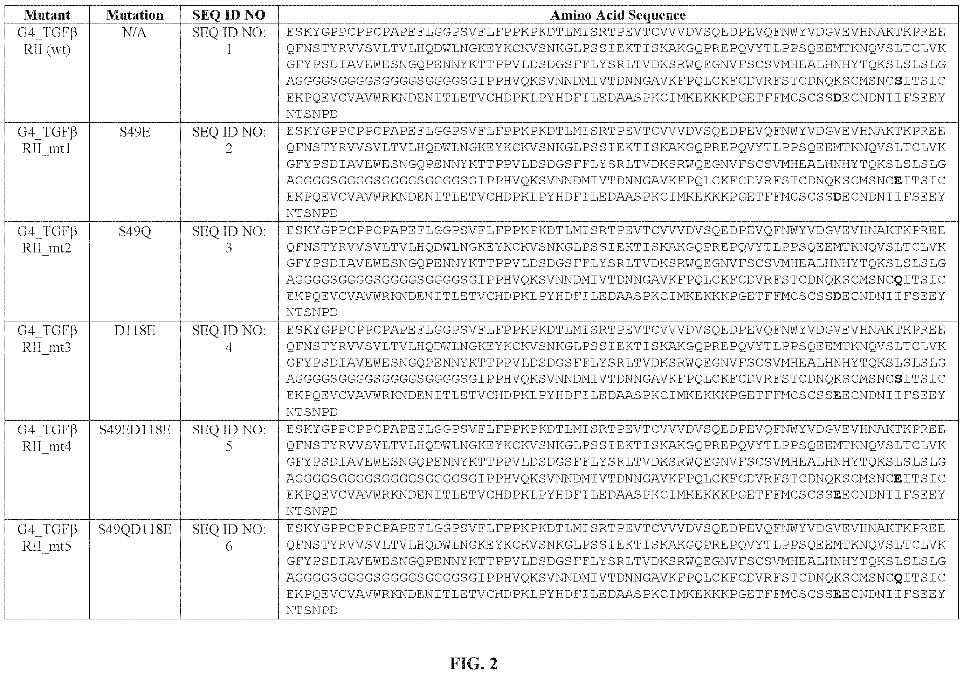

Engineered TGFβRII variants (G4_TGFβRII proteins) were designed by linking mutated TGFβRII extracellular domains to a Fc region of IgG4 according to the sequences listed in **FIG. 2.**

### Example 2. Production and CMC analysis for G4_TGFβRII proteins

G4_TGFβRII proteins were expressed according to the sequences listed in **FIG. 2**.

The expressed proteins were purified by a protein A column, followed by HPLC-SEC (high-performance liquid chromatography coupled with size exclusion chromatography; Agilent), and the percentage of high molecular weight peaks (HMW%), the percentage of the major peak (Major%), and the percentage of low molecular weight peaks (LMW%) were measured. The amino acid sequences were analyzed using the deimmunization tool (Immune Epitope Database And Analysis Resource; Dhanda et al. "Development of a strategy and computational application to select candidate protein analogues with reduced HLA binding and immunogenicity." Immunology 153.1 (2018): 118-132) to identify immunogenic regions. No immunogenicity was identified.

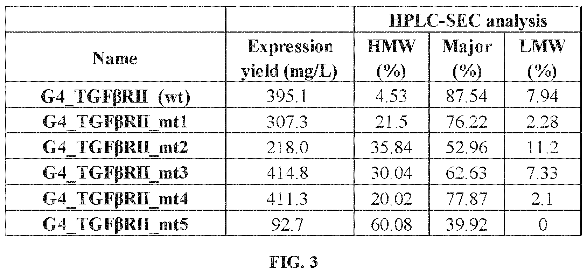

**FIG. 3** is a summary of production and CMC analysis results. HMW% indicated the percentage of high-molecular weight species in the sample after protein A purification. Major% indicated the percentage of major molecular weight species in the sample after protein A purification. LMW% indicated the percentage of low-molecular weight species in the sample after protein A purification.

The expression yield was determined by high sensitivity (hs) IgG Assay kit (4BioCell, Cat. no.: 200112, 800312) using CuBiAnXC (OPTOCELL technology). The HPLC SEC profiles was determined and analyzed by Waters E2695 Separations Module (Waters Corporation) with AdvanceBio SEC 2.7 um Columns (Agilent Technologies, Inc.).

The data in **FIG. 3** showed that G4_TGFβRII_mt5 has relatively lower expression yeild and lower major molecular weight species. G4_TGFβRII _mt5 was not used in further in vitro functional analysis.

### Example 3. ELISA binding assays

To determine the binding ability of G4_TGFβRII proteins to human TGFβ, a binding titration ELISA assay was performed using of the G4_TGFβRII proteins at indicated concentrations (1.1 pM, 6.4 pM, 38.6 pM, 231.5 pM, 1.4 nM, 8.3 nM, 50 nM, or 300 nM). G4_TGFβRII (wt) was used as a positive control. IgG4 isotype control was used as a negative control. A 96-well EIA microplate was coated with 0.5 μg/ml human TGFβ (TGFβ1, TGFβ2, or TGFβ3) overnight at 4°C. After blocking with 1xPBS containing 5% skim milk, diluted G4_TGFβRII proteins (mt1, mt2, mt3, mt4) were added and incubated at room temperature (RT) for 1 hour. The unbound proteins were removed by washing the wells with 1xPBST (1xPBS containing 0.05% Tween 20) three times. A HRP-conjugated secondary antibody (1:5000) was added to the wells at room temperature (RT) for 1 hour. After the incubation, excess secondary antibodies were removed by washing the wells with 1xPBST three times. Finally, 3,3',5,5'-Tetramethylbenzidine (TMB) was added for color development. The reaction was stopped and HRP activity was measured using a spectrophotometer at 450 nm.

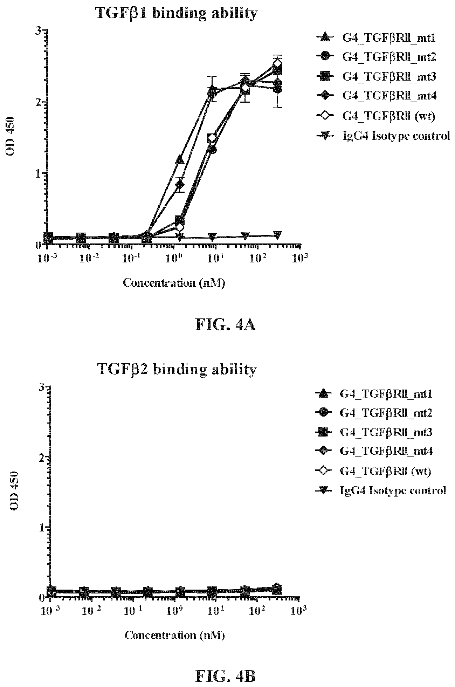

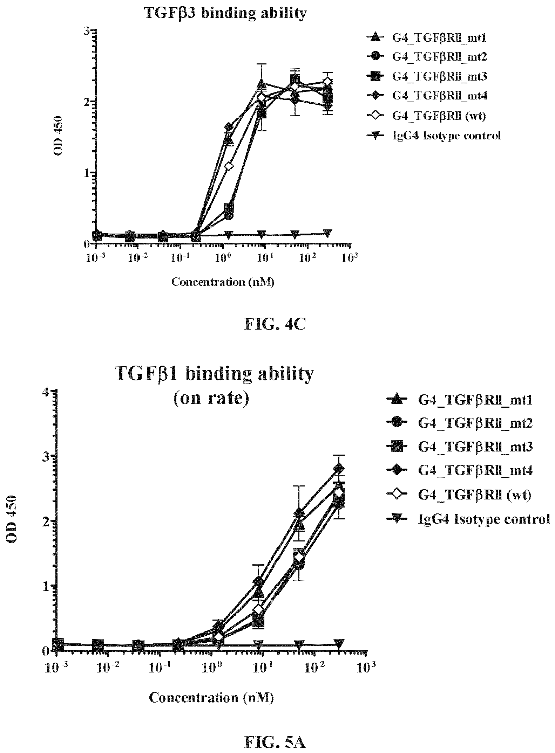

**FIGS. 4A-4C** show the binding affinities between G4_TGFβRII proteins and human TGFβ, as measured by ELISA. The data showed that all tested G4_TGFβRII proteins can bind to TGFβ1 and TGFβ3. As shown in **FIG. 4A**, for TGFβ1, G4_TGFβRII_mt1 and G4_TGFβRII_mt4 showed better binding affinities compared to G4_TGFβRII (wt). G4_TGFβRII_mt2, G4_TGFβRII_mt3, and G4_TGFβRII (wt) showed similar binding affinities. As shown in **FIG. 4C**, for TGFβ3, G4_TGFβRII_mt1 and G4_TGFβRII_mt4 showed better binding affinities than G4_TGFβRII (wt). G4_TGFβRII_mt2 and G4_TGFβRII_mt3 showed worse binding affinities than G4_TGFβRII (wt). As shown in **FIG. 4B**, none of the tested TGFβRII proteins showed binding to TGFβ2.

### Example 4. ELISA binding assays (on-rate and off-rate)

To determine the on-rate and off-rate binding ability of G4_TGFβRII proteins to human TGFβ1, a binding titration ELISA assay was performed using of the G4_TGFβRII proteins at indicated concentrations (1.1 pM, 6.4 pM, 38.6 pM, 231.5 pM, 1.4 nM, 8.3 nM, 50 nM, or 300 nM). G4_TGFβRII (wt) was used as a positive control. IgG4 isotype control was used as a negative control. For on-rate binding ability, a 96-well EIA microplate was coated with 0.5 μg/ml human TGFβ (TGFβ1) overnight at 4°C. After blocking with 1xPBS containing 5% skim milk, diluted G4_TGFβRII proteins were added and incubated at room temperature (RT) for 5 min. The unbound proteins were removed by washing the wells with 1xPBST (1xPBS containing 0.1% Tween 20) three times. A HRP-conjugated secondary antibody (1:5000) was added to the wells at RT for 1 hour. After the incubation, excess secondary antibodies were removed by washing the wells with 1xPBST three times. Finally, 3,3',5,5'-Tetramethylbenzidine (TMB) was added for color development. The reaction was stopped and HRP activity was measured using a spectrophotometer at 450 nm.

For off-rate binding ability, a 96-well EIA microplate was coated with 0.5 μg/ml human TGFβ (TGFβ1) overnight at 4°C. After blocking with 1 × PBS containing 5% skim milk, diluted G4_TGFβRII proteins were added and incubated at room temperature (RT) for I hour. The unbound proteins were removed by washing the wells with 1xPBST (1× PBS containing 0.1% Tween 20) at 800 rpm shaker for 2 hours with changing washing buffer every half hour. A HRP-conjugated secondary antibody (1:5000) was added to the wells at RT for 1 hour. After the incubation, excess secondary antibodies were removed by washing the wells with 1xPBST three times. Finally, 3,3',5,5'-Tetramethylbenzidine (TMB) was added for color development. The reaction was stopped and HRP activity was measured using a spectrophotometer at 450 nm.

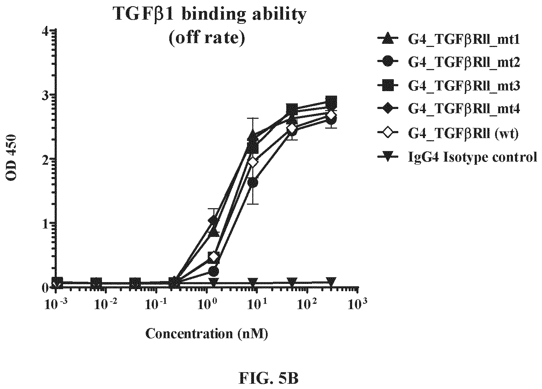

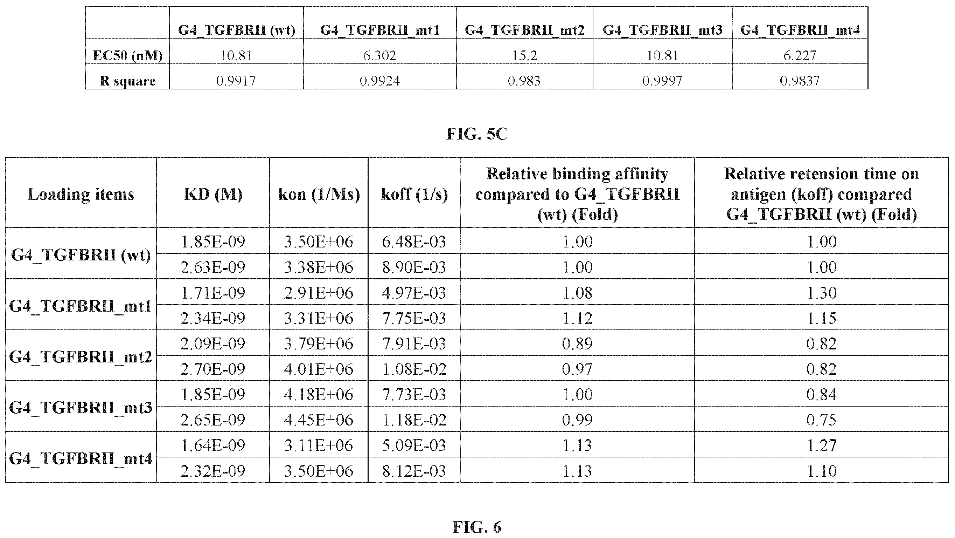

**FIG. 5A** shows the on rate binding ability between G4_TGFβRII proteins and human TGFβ1, as measured by ELISA. **FIG. 5B** shows the off rate binding ability between G4_TGFβRII proteins and human TGFβ1, as measured by ELISA. As shown in **FIG. 5A**, for on rate, G4_TGFβRII _mt1 and G4_TGFβRII _mt4 showed better binding abilities than G4_TGFβRII (wt). G4_TGFβRII_mt2, G4_TGFβRII_mt3 and G4_TGFβRII (wt) showed similar binding abilities. As shown in **FIG. 5B**, for off rate, G4_TGFβRII_mt1 and m4 showed better binding abilities than G4_TGFβRII (wt). G4_TGFβRII_mt3 and G4_TGFβRII (wt) showed similar binding abilities. G4_TGFβRII_mt2 showed worse binding ability than G4_TGFβRII (wt). **FIG. 5C** shows EC50 of the on rate binding.

### Example 5. Binding affinity determination using BLI

TGFβ binding affinity was further determined by Bio-Layer Interferometry (BLI) using Octet Red96 (Forebio). G4_TGFBRII proteins were immobilized on anti-Human IgG Fc capture (AHC) Biosensors (Fortebio, cat. No. 18-5060). Serial diluted TGFβ1 or TGFβ3 from 18.8 nM to 75 nM in the assay buffer (1xPBS containing 0.05% Tween-20 with 0.1%BSA at pH7.4) were used as analytes for the G4_TGFBRII proteins. Binding kinetics were evaluated using the 1:1 Langmuir binding model in Fortebio Data Analysis 11.0 Software.

The results of the BLI binding assay are shown in **FIG. 6**. As shown in **FIG. 6**, G4_TGFβRII_mt1 and G4_TGFβRII_mt4 showed the best TGFβ1 and TGFβ3 binding affinities. Further, G4_TGFβRII_mt1 and G4_TGFβRII_mt4 showed the best TGFβ1 and TGFβ3 retention time (koff).

### Example 6. Inhibition of TGFβ-induced smad2 reporter activity

The ability of G4_TGFβRII proteins to inhibit TGFβ1-, TGFβ2-, or TGFβ3-induced smad2 reporter activity was determined. The G4 TGFβRII proteins were serially diluted (5-fold) to final concentrations of 6.4 pM, 32 pM, 160 pM, 0.8 nM, 4 nM, 20 nM, 100 nM, and 500 nM. G4_TGFβRII (wt) was used as a positive control. IgG4 isotype control was used as a negative control. $4 \times 10^3$ transfected HEK293T cells expressing a smad2 reporter was incubated with the diluted G4_TGFβRII proteins, together with 20 ng/mL TGFβ1, TGFβ2, or TGFβ3, respectively. After a 24-hour incubation at 37°C in a 5% $CO_2$ incubator, luminescence signals were detected by Varioskan™ LUX multimode microplate reader (Thermo).

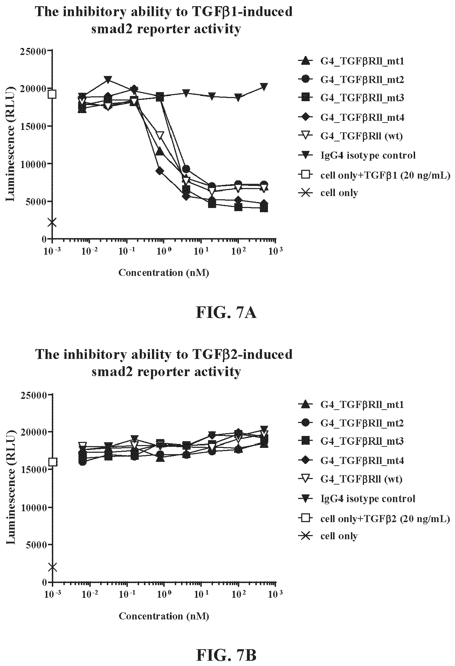

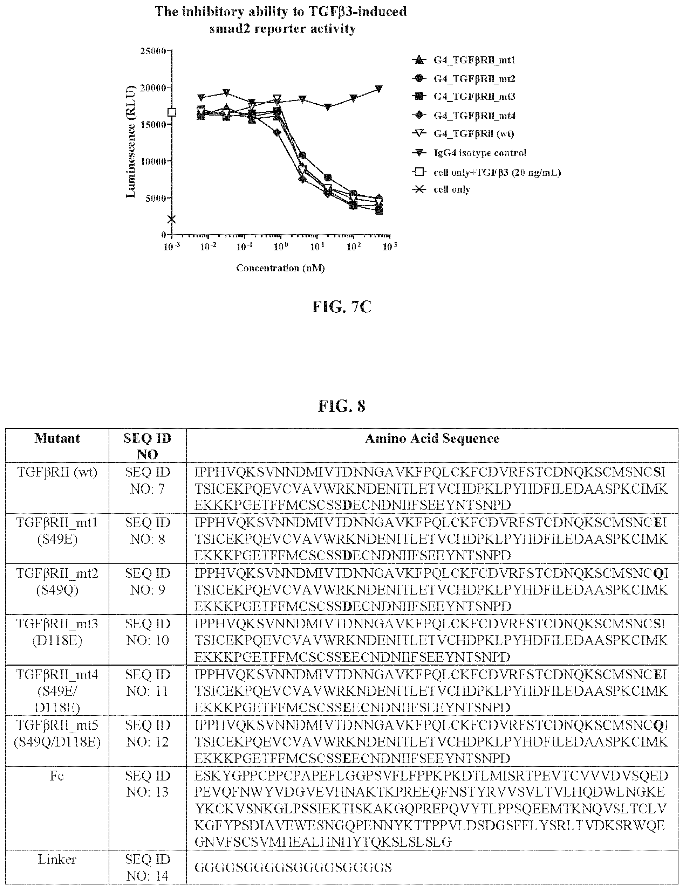

The results from the smad2 reporter activity assay are shown in **FIGs. 7A-7C**. As shown in **FIG. 7A**, all four G4-TGFβRII proteins can inhibit TGFβ1-mediated smad2 reporter activity. G4_TGFβRII_mt4 and G4_TGFβRII_mt1 showed better inhibitory effects than G4_TGFβRII (wt). G4_TGFβRII_mt2 and G4_TGFβRII_mt3 showed worse inhibitory effect than G4_TGFβRII (wt). As shown in **FIG. 7B**, none of the G4-TGFβRII proteins inhibited TGFβ2-induced smad2 reporter activity. As shown in **FIG. 7C**, all four G4-TGFβRII proteins can inhibit TGFβ3-mediated smad2 reporter activity. G4_TGFβRII_mt4, G4_TGFβRII_mt1 and G4_TGFβRII_mt3 showed better inhibitory effects than G4_TGFβRII (wt). G4_TGFβRII_mt2 showed worse inhibitory effects than G4_TGFβRII (wt).

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EMBODIMENTS

Although the present invention is defined in the claims, it should be understood that the present invention can also (alternatively) be defined in accordance with the following embodiments:

1. An engineered TGFβRII polypeptide comprising an amino acid sequence that is at least 80% identical to SEQ ID NO: 7, wherein the engineered TGFβRII polypeptide comprises one or more amino acid mutations at positions corresponding to S49 and D118 of SEQ ID NO: 7.

2. The engineered TGFβRII polypeptide of embodiment 1, wherein the engineered TGFβRII polypeptide comprises one or more of the following:

    (a) the amino acid that corresponds to S49 of SEQ ID NO: 7 is G, A, L, M, F, W, K, Q, E, P, V, I, C, Y, H, R, N, D, or T; and
    (b) the amino acid that corresponds to D118 of SEQ ID NO: 7 is G, A, L, M, F, W, K, Q, E, S, P, V, I, C, Y, H, R, N, or T.

3. The engineered TGFβRII polypeptide of embodiment 1 or 2, wherein the amino acid that corresponds to S49 of SEQ ID NO: 7 is E or Q.

4. The engineered TGFβRII polypeptide any one of embodiments 1-3, wherein the amino acid that corresponds to D118 of SEQ ID NO: 7 is E.

5. The engineered TGFβRII polypeptide of any one of embodiments 1-4, wherein the amino acid that corresponds to S49 of SEQ ID NO: 7 is E.

6. The engineered TGFβRII polypeptide of any one of embodiments 1-4, wherein the amino acid that corresponds to position 32 of SEQ ID NO: 7 is D.

7. The engineered TGFβRII polypeptide of any one of embodiments 1-6, wherein the amino acid that corresponds to position 119 of SEQ ID NO: 7 is E.

8. The engineered TGFβRII polypeptide of any one of embodiments 1-7, wherein the engineered TGF-βRII polypeptide comprises an amino acid sequence that is at least 85%, 90%, 95%, 95%, 96%, 97%, 98%, 99%, or 100% identical to any one of SEQ ID NOs: 7-12.

9. The engineered TGFβRII polypeptide of embodiment 8, wherein the engineered TGFβRII polypeptide comprises an amino acid sequence that is at least 90% identical to SEQ ID NO: 8.

10. The engineered TGFβRII polypeptide of embodiment 8, wherein the engineered TGFβRII polypeptide comprises an amino acid sequence that is at least 90% identical to SEQ ID NO: 9.

11. The engineered TGFβRII polypeptide of embodiment 8, wherein the engineered TGFβRII polypeptide comprises an amino acid sequence that is at least 90% identical to SEQ ID NO: 10.

12. The engineered TGFβRII polypeptide of embodiment 8, wherein the engineered TGFβRII polypeptide comprises an amino acid sequence that is at least 90% identical to SEQ ID NO: 11.

13. The engineered TGFβRII polypeptide of embodiment 8, wherein the engineered TGFβRII polypeptide comprises an amino acid sequence that is at least 90% identical to SEQ ID NO: 12.

14. The engineered TGFβRII polypeptide of any one of embodiments 1-13, wherein the off rate between the engineered TGFβRII polypeptide and TGFβ1 is lower than the off rate between wildtype TGFβRII and TGFβ1.

15. The engineered TGFβRII polypeptide of any one of embodiments 1-14, wherein the off rate between the engineered TGFβRII polypeptide and TGFβ3 is lower than the off rate between wildtype TGFβRII and TGFβ3.

16. The engineered TGFβRII polypeptide of any one of embodiments 1-15, wherein the engineered TGF-βRII polypeptide does not bind to TGFβ2.

17. The engineered TGFβRII polypeptide of any one of embodiments 1-16, wherein the engineered TGF-βRII polypeptide interacts with R94, R25, K37, and/or K31 on TGFβ1.

18. The engineered TGFβRII polypeptide of any one of embodiments 1-17, wherein the engineered TGF-βRII polypeptide interacts with R94, R25, K37, and/or K31 on TGFβ3.

19. The engineered TGFβRII polypeptide of any one of embodiments 1-18, wherein the engineered TGF-βRII polypeptide interacts with R94, R25, K37, and/or K31 on TGFβ1 via hydrogen bond interactions.

20. The engineered TGFβRII polypeptide of any one of embodiments 1-19, wherein the engineered TGF-βRII polypeptide interacts with R94, R25, K37, and/or K31 on TGFβ3 via hydrogen bond interactions.

21. The engineered TGFβRII polypeptide of any one of embodiments 1-20, wherein the engineered TGF-βRII polypeptide can capture TGFβ (e.g., TGFβ1) and thereby improve tumor microenvironment.

22. The engineered TGFβRII polypeptide of any one of embodiments 1-21, wherein the engineered TGF-βRII polypeptide can block or interfere the binding between TGFβ (e.g., TGFβ1) and TGFβRII.

23. The engineered TGFβRII polypeptide of any one of embodiments 1-22, wherein the engineered TGF-βRII polypeptide can block or interfere the binding between TGFβ (e.g., TGFβ1) and TGFβRII expressed on the surface of a tumor cells, fibroblasts, or immune cells.

24. The engineered TGFβRII polypeptide of any one of embodiments 1-23, wherein the engineered TGF-βRII polypeptide further comprises a CH2 domain and a CH3 domain.

25. The engineered TGFβRII polypeptide of embodiment 24, wherein the engineered TGFβRII polypeptide further comprises a hinge region.

26. The engineered TGFβRII polypeptide of embodiment 24 or 25, wherein the CH2 domain is an IgG CH2 domain and the CH3 domain is an IgG CH3 domain.

27. The engineered TGFβRII polypeptide of any one of embodiments 24-26, wherein the engineered TGFβRII polypeptide are linked to either (a) the N-terminus of the hinge region or CH2 domain, or (b) the C-terminus of the CH3 domain, optionally through a linker sequence.

28. The engineered TGFβRII polypeptide of any one of embodiments 24-27, wherein the engineered TGFβRII polypeptide comprises an amino acid sequence that is identical to any one of SEQ ID NO: 1-6.

29. A protein construct comprising the engineered TGFβRII polypeptide of any one of embodiments 1-28.

30. The protein construct of embodiment 29, wherein the protein construct comprises two or more engineered TGFβRII polypeptides.

31. The protein construct of embodiment 30, wherein at least two of the engineered TGFβRII polypeptides are identical.

32. The protein construct of embodiment 30, wherein at least two of the engineered TGFβRII polypeptides are different.

33. The protein construct of any one of embodiments 29-32, further comprising an Fc region.

34. The protein construct of embodiment 33, wherein the Fc region is an IgG4 Fc region.

35. The protein construct of embodiment 34, wherein the Fc region is an IgG1 Fc region (e.g., with LALA mutations or LALA-PG mutations).

36. The protein construct of any one of embodiments 33-35, wherein the engineered TGFβRII polypeptide is linked to the C-terminus of the Fc region.

37. The protein construct of any one of embodiments 33-35, wherein the engineered TGFβRII polypeptide is linked to the C-terminus of the Fc region via a peptide linker.

38. A protein construct comprising

a first fusion polypeptide comprising the engineered TGFβRII polypeptide of any one of embodiments 1-28, a first CH2 domain, and a first CH3 domain; and
a second fusion polypeptide comprising a second CH2 domain, and a second CH3 domain, wherein the first fusion polypeptide and the second fusion polypeptide associate with each other, forming a dimer.

39. The protein construct of embodiment 38, wherein the second fusion polypeptide further comprises a second engineered TGFβRII polypeptide.

40. A pharmaceutical composition comprising the engineered TGFβRII polypeptide of any one of embodiments 1-28 or the protein construct of any one of embodiments 29-39; and
a pharmaceutically acceptable carrier.

41. A nucleic acid encoding the engineered TGFβRII polypeptide of any one of embodiments 1-28 or the protein construct of any one of embodiments 29-39.

42. A vector comprising the nucleic acid of embodiment 41.

43. A cell comprising the nucleic acid of embodiment 41 or the vector of embodiment 42.

44. The cell of embodiment 43, wherein the cell is a CHO cell.

45. A method of producing an engineered TGFβRII polypeptide or a protein construct comprising the engineered TGFβRII polypeptide, the method comprising

(a) culturing the cell of embodiment 43 or 44 under conditions sufficient for the cell to produce the engineered TGFβRII polypeptide or the protein construct; and
(b) collecting the engineered TGFβRII polypeptide or the protein construct produced by the cell.

46. A method of treating a subject having cancer, the method comprising administering a therapeutically effective amount of a composition comprising the engineered TGFβRII polypeptide of any one of embodiments 1-28 or the protein construct of any one of embodiments 29-39, to the subject.

47. The method of embodiment 46, wherein the subject has a solid tumor or a hematologic cancer.

48. The method of embodiment 46, wherein the cancer is breast cancer, ovarian cancer, glioma, colon cancer, hepatocellular carcinoma, prostate cancer, renal cell carcinoma, melanoma, lung cancer, pancreatic cancer, or hepatoma.

49. A method of decreasing the rate of tumor growth, the method comprising contacting a tumor cell with an effective amount of a composition comprising the engineered TGFβRII polypeptide of any one of embodiments 1-28 or the protein construct of any one of embodiments 29-39.

50. A method of killing a tumor cell, the method comprising
contacting a tumor cell with an effective amount of a composition comprising the engineered TGFβRII polypeptide of any one of embodiments 1-28 or the protein construct of any one of embodiments 29-39.

**Claims**

1. An engineered TGFβRII polypeptide comprising an amino acid sequence that is at least 80% identical to SEQ ID NO: 7, wherein the engineered TGFβRII polypeptide comprises one or more amino acid mutations at positions corresponding to S49 and D118 of SEQ ID NO: 7.

2. The engineered TGFβRII polypeptide of claim 1, wherein the engineered TGFβRII polypeptide comprises one or more of the following:

(a) the amino acid that corresponds to S49 of SEQ ID NO: 7 is G, A, L, M, F, W, K, Q, E, P, V, I, C, Y, H, R, N, D, or T; and
(b) the amino acid that corresponds to D118 of SEQ ID NO: 7 is G, A, L, M, F, W, K, Q, E, S, P, V, I, C, Y, H, R, N, or T.

3. The engineered TGFβRII polypeptide of claim 1 or 2, wherein the amino acid that corresponds to S49 of SEQ ID NO: 7 is E or Q, and/or wherein the amino acid that corresponds to D118 of SEQ ID NO: 7 is E, and/or wherein the amino acid that corresponds to S49 of SEQ ID NO: 7 is E, or wherein the amino acid that corresponds to position 32 of SEQ ID NO: 7 is D, and/or wherein the amino acid that corresponds to position 119 of SEQ ID NO: 7 is E.

4. The engineered TGFβRII polypeptide of any one of claims 1-3, wherein the engineered TGFβRII polypeptide comprises an amino acid sequence that is at least 85%, 90%, 95%, 95%, 96%, 97%, 98%, 99%, or 100% identical to any one of SEQ ID NOs: 7-12.

5. The engineered TGFβRII polypeptide of claim 4, wherein the engineered TGFβRII polypeptide comprises an amino acid sequence that is at least 90% identical to SEQ ID NO: 8, or wherein the engineered TGFβRII polypeptide comprises an amino acid sequence that is at least 90% identical to SEQ ID NO: 9, or wherein the engineered TGFβRII polypeptide comprises an amino acid sequence that is at least 90% identical to SEQ ID NO: 10, or wherein the engineered TGFβRII polypeptide comprises an amino acid sequence that is at least 90% identical to SEQ ID NO: 11, or wherein the engineered TGFβRII polypeptide comprises an amino acid sequence that is at least 90% identical to SEQ ID NO: 12.

6. The engineered TGFβRII polypeptide of any one of claims 1-5, wherein the off rate between the engineered TGFβRII polypeptide and TGFβ1 is lower than the off rate between wildtype TGFβRII and TGFβ1, and/or wherein the off rate between the engineered TGFβRII polypeptide and TGFβ3 is lower than the off rate between wildtype TGFβRII and TGFβ3, and/or wherein the engineered TGFβRII polypeptide does not bind to TGFβ2, and/or wherein the engineered TGFβRII polypeptide interacts with R94, R25, K37, and/or K31 on TGFβ1, and/or wherein the engineered TGFβRII polypeptide interacts with R94, R25, K37, and/or K31 on TGFβ3, and/or wherein the engineered TGFβRII polypeptide interacts with R94, R25, K37, and/or K31 on TGFβ1 via hydrogen bond interactions, and/or wherein the engineered TGFβRII polypeptide interacts with R94, R25, K37, and/or K31 on TGFβ3 via hydrogen bond interactions, and/or wherein the engineered TGFβRII polypeptide can capture TGFβ (e.g., TGFβ1) and thereby improve tumor microenvironment, and/or wherein the engineered TGFβRII polypeptide can block or interfere the binding between TGFβ (e.g., TGFβ1) and TGFβRII, and/or wherein the engineered TGFβRII polypeptide can block or interfere the binding between TGFβ (e.g., TGFβ1) and TGFβRII expressed on the surface of a tumor cells, fibroblasts, or immune cells.

7. The engineered TGFβRII polypeptide of any one of claims 1-6, wherein the engineered TGFβRII polypeptide further comprises a CH2 domain and a CH3 domain, optionally wherein the engineered TGFβRII polypeptide further comprises a hinge region, optionally wherein the CH2 domain is an IgG CH2 domain and the CH3 domain is an IgG CH3 domain, optionally wherein the engineered TGFβRII polypeptide are linked to either (a) the N-terminus of the hinge region or CH2 domain, or (b) the C-terminus of the CH3 domain, optionally through a linker sequence, optionally wherein the engineered TGFβRII polypeptide comprises an amino acid sequence that is identical to any one of SEQ ID NO: 1-6.

8. A protein construct comprising the engineered TGFβRII polypeptide of any one of claims 1-7.

9. The protein construct of claim 8, wherein the protein construct comprises two or more engineered TGFβRII polypeptides, optionally wherein at least two of the engineered TGFβRII polypeptides are identical, optionally wherein at least two of the engineered TGFβRII polypeptides are different, and/or further comprising an Fc region, optionally wherein the Fc region is an IgG4 Fc region, optionally wherein the Fc region is an IgG1 Fc region (e.g., with LALA mutations or LALA-PG mutations), optionally wherein the engineered TGFβRII polypeptide is linked to the C-terminus of the Fc region, or optionally wherein the engineered TGFβRII polypeptide is linked to the C-terminus of the Fc region via a peptide linker.

10. A protein construct comprising

a first fusion polypeptide comprising the engineered TGFβRII polypeptide of any one of claims 1-7, a first CH2 domain, and a first CH3 domain; and
a second fusion polypeptide comprising a second CH2 domain, and a second CH3 domain, wherein the first fusion polypeptide and the second fusion polypeptide associate with each other, forming a dimer, optionally wherein the second fusion polypeptide further comprises a second engineered TGFβRII polypeptide.

11. A pharmaceutical composition comprising the engineered TGFβRII polypeptide of any one of claims 1-7 or the protein construct of any one of claims 8-10; and

a pharmaceutically acceptable carrier.

12. A nucleic acid encoding the engineered TGFβRII polypeptide of any one of claims 1-7 or the protein construct of any one of claims 8-10.

13. A cell comprising the nucleic acid of claim 12, optionally wherein the cell is a CHO cell.

14. A method of producing an engineered TGFβRII polypeptide or a protein construct comprising the engineered TGFβRII polypeptide, the method comprising

(a) culturing the cell of claim 13 under conditions sufficient for the cell to produce the engineered TGFβRII polypeptide or the protein construct; and
(b) collecting the engineered TGFβRII polypeptide or the protein construct produced by the cell.

15. A composition comprising the engineered TGFβRII polypeptide of any one of claims 1-7 or the protein construct of any one of claims 8-10 for use in a method of treating a subject having cancer, the method comprising administering a therapeutically effective amount of said composition to the subject, optionally wherein the subject has a solid tumor or a hematologic cancer, or optionally wherein the cancer is breast cancer, ovarian cancer, glioma, colon cancer, hepatocellular carcinoma, prostate cancer, renal cell carcinoma, melanoma, lung cancer, pancreatic cancer, or hepatoma.

FIG. 1A

FIG. 1B

| Mutant | Mutation | SEQ ID NO | Amino Acid Sequence |
|---|---|---|---|
| G4_TGFβ RII (wt) | N/A | SEQ ID NO: 1 | ESKYGPPCPPCPAPEFLGGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEALHNHYTQKSLSLSLG AGGGGSGGGGSGGGGSGGGGSGIPPHVQKSVNNDMIVTDNNGAVKFPQLCKFCDVRFSTCDNQKSCMSNC**S**ITSIC EKPQEVCVAVWRKNDENITLETVCHDPKLPYHDFILEDAASPKCIMKEKKKPGETFFMCSCSS**D**ECNDNIIFSEEY NTSNPD |
| G4_TGFβ RII_mt1 | S49E | SEQ ID NO: 2 | ESKYGPPCPPCPAPEFLGGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEALHNHYTQKSLSLSLG AGGGGSGGGGSGGGGSGGGGSGIPPHVQKSVNNDMIVTDNNGAVKFPQLCKFCDVRFSTCDNQKSCMSNC**E**ITSIC EKPQEVCVAVWRKNDENITLETVCHDPKLPYHDFILEDAASPKCIMKEKKKPGETFFMCSCSS**D**ECNDNIIFSEEY NTSNPD |
| G4_TGFβ RII_mt2 | S49Q | SEQ ID NO: 3 | ESKYGPPCPPCPAPEFLGGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEALHNHYTQKSLSLSLG AGGGGSGGGGSGGGGSGGGGSGIPPHVQKSVNNDMIVTDNNGAVKFPQLCKFCDVRFSTCDNQKSCMSNC**Q**ITSIC EKPQEVCVAVWRKNDENITLETVCHDPKLPYHDFILEDAASPKCIMKEKKKPGETFFMCSCSS**D**ECNDNIIFSEEY NTSNPD |
| G4_TGFβ RII_mt3 | D118E | SEQ ID NO: 4 | ESKYGPPCPPCPAPEFLGGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEALHNHYTQKSLSLSLG AGGGGSGGGGSGGGGSGGGGSGIPPHVQKSVNNDMIVTDNNGAVKFPQLCKFCDVRFSTCDNQKSCMSNC**S**ITSIC EKPQEVCVAVWRKNDENITLETVCHDPKLPYHDFILEDAASPKCIMKEKKKPGETFFMCSCSS**E**ECNDNIIFSEEY NTSNPD |
| G4_TGFβ RII_mt4 | S49ED118E | SEQ ID NO: 5 | ESKYGPPCPPCPAPEFLGGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEALHNHYTQKSLSLSLG AGGGGSGGGGSGGGGSGGGGSGIPPHVQKSVNNDMIVTDNNGAVKFPQLCKFCDVRFSTCDNQKSCMSNC**E**ITSIC EKPQEVCVAVWRKNDENITLETVCHDPKLPYHDFILEDAASPKCIMKEKKKPGETFFMCSCSS**E**ECNDNIIFSEEY NTSNPD |
| G4_TGFβ RII_mt5 | S49QD118E | SEQ ID NO: 6 | ESKYGPPCPPCPAPEFLGGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEALHNHYTQKSLSLSLG AGGGGSGGGGSGGGGSGGGGSGIPPHVQKSVNNDMIVTDNNGAVKFPQLCKFCDVRFSTCDNQKSCMSNC**Q**ITSIC EKPQEVCVAVWRKNDENITLETVCHDPKLPYHDFILEDAASPKCIMKEKKKPGETFFMCSCSS**E**ECNDNIIFSEEY NTSNPD |

FIG. 2

| Name | Expression yield (mg/L) | HPLC-SEC analysis | | |
|---|---|---|---|---|
| | | HMW (%) | Major (%) | LMW (%) |
| G4_TGFβRII (wt) | 395.1 | 4.53 | 87.54 | 7.94 |
| G4_TGFβRII_mt1 | 307.3 | 21.5 | 76.22 | 2.28 |
| G4_TGFβRII_mt2 | 218.0 | 35.84 | 52.96 | 11.2 |
| G4_TGFβRII_mt3 | 414.8 | 30.04 | 62.63 | 7.33 |
| G4_TGFβRII_mt4 | 411.3 | 20.02 | 77.87 | 2.1 |
| G4_TGFβRII_mt5 | 92.7 | 60.08 | 39.92 | 0 |

FIG. 3

**FIG. 4A**

**FIG. 4B**

TGFβ3 binding ability

FIG. 4C

TGFβ1 binding ability
(on rate)

FIG. 5A

FIG. 5B

|  | G4_TGFBRII (wt) | G4_TGFBRII_mt1 | G4_TGFBRII_mt2 | G4_TGFBRII_mt3 | G4_TGFBRII_mt4 |
|---|---|---|---|---|---|
| EC50 (nM) | 10.81 | 6.302 | 15.2 | 10.81 | 6.227 |
| R square | 0.9917 | 0.9924 | 0.983 | 0.9997 | 0.9837 |

**FIG. 5C**

| Loading items | KD (M) | kon (1/Ms) | koff (1/s) | Relative binding affinity compared to G4_TGFBRII (wt) (Fold) | Relative retension time on antigen (koff) compared G4_TGFBRII (wt) (Fold) |
|---|---|---|---|---|---|
| G4_TGFBRII (wt) | 1.85E-09 | 3.50E+06 | 6.48E-03 | 1.00 | 1.00 |
|  | 2.63E-09 | 3.38E+06 | 8.90E-03 | 1.00 | 1.00 |
| G4_TGFBRII_mt1 | 1.71E-09 | 2.91E+06 | 4.97E-03 | 1.08 | 1.30 |
|  | 2.34E-09 | 3.31E+06 | 7.75E-03 | 1.12 | 1.15 |
| G4_TGFBRII_mt2 | 2.09E-09 | 3.79E+06 | 7.91E-03 | 0.89 | 0.82 |
|  | 2.70E-09 | 4.01E+06 | 1.08E-02 | 0.97 | 0.82 |
| G4_TGFBRII_mt3 | 1.85E-09 | 4.18E+06 | 7.73E-03 | 1.00 | 0.84 |
|  | 2.65E-09 | 4.45E+06 | 1.18E-02 | 0.99 | 0.75 |
| G4_TGFBRII_mt4 | 1.64E-09 | 3.11E+06 | 5.09E-03 | 1.13 | 1.27 |
|  | 2.32E-09 | 3.50E+06 | 8.12E-03 | 1.13 | 1.10 |

**FIG. 6**

**The inhibitory ability to TGFβ1-induced
smad2 reporter activity**

Legend:
- G4_TGFβRII_mt1
- G4_TGFβRII_mt2
- G4_TGFβRII_mt3
- G4_TGFβRII_mt4
- G4_TGFβRII (wt)
- IgG4 isotype control
- cell only+TGFβ1 (20 ng/mL)
- cell only

**FIG. 7A**

**The inhibitory ability to TGFβ2-induced
smad2 reporter activity**

Legend:
- G4_TGFβRII_mt1
- G4_TGFβRII_mt2
- G4_TGFβRII_mt3
- G4_TGFβRII_mt4
- G4_TGFβRII (wt)
- IgG4 isotype control
- cell only+TGFβ2 (20 ng/mL)
- cell only

**FIG. 7B**

The inhibitory ability to TGFβ3-induced smad2 reporter activity

FIG. 7C

FIG. 8

| Mutant | SEQ ID NO | Amino Acid Sequence |
|---|---|---|
| TGFβRII (wt) | SEQ ID NO: 7 | IPPHVQKSVNNDMIVTDNNGAVKFPQLCKFCDVRFSTCDNQKSCMSNCSITSICEKPQEVCVAVWRKNDENITLETVCHDPKLPYHDFILEDAASPKCIMKEKKKPGETFFMCSCSS**D**ECNDNIIFSEEYNTSNPD |
| TGFβRII_mt1 (S49E) | SEQ ID NO: 8 | IPPHVQKSVNNDMIVTDNNGAVKFPQLCKFCDVRFSTCDNQKSCMSNC**E**ITSICEKPQEVCVAVWRKNDENITLETVCHDPKLPYHDFILEDAASPKCIMKEKKKPGETFFMCSCSS**D**ECNDNIIFSEEYNTSNPD |
| TGFβRII_mt2 (S49Q) | SEQ ID NO: 9 | IPPHVQKSVNNDMIVTDNNGAVKFPQLCKFCDVRFSTCDNQKSCMSNC**Q**ITSICEKPQEVCVAVWRKNDENITLETVCHDPKLPYHDFILEDAASPKCIMKEKKKPGETFFMCSCSS**D**ECNDNIIFSEEYNTSNPD |
| TGFβRII_mt3 (D118E) | SEQ ID NO: 10 | IPPHVQKSVNNDMIVTDNNGAVKFPQLCKFCDVRFSTCDNQKSCMSNCSITSICEKPQEVCVAVWRKNDENITLETVCHDPKLPYHDFILEDAASPKCIMKEKKKPGETFFMCSCSS**E**ECNDNIIFSEEYNTSNPD |
| TGFβRII_mt4 (S49E/D118E) | SEQ ID NO: 11 | IPPHVQKSVNNDMIVTDNNGAVKFPQLCKFCDVRFSTCDNQKSCMSNC**E**ITSICEKPQEVCVAVWRKNDENITLETVCHDPKLPYHDFILEDAASPKCIMKEKKKPGETFFMCSCSS**E**ECNDNIIFSEEYNTSNPD |
| TGFβRII_mt5 (S49Q/D118E) | SEQ ID NO: 12 | IPPHVQKSVNNDMIVTDNNGAVKFPQLCKFCDVRFSTCDNQKSCMSNC**Q**ITSICEKPQEVCVAVWRKNDENITLETVCHDPKLPYHDFILEDAASPKCIMKEKKKPGETFFMCSCSS**E**ECNDNIIFSEEYNTSNPD |
| Fc | SEQ ID NO: 13 | ESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLG |
| Linker | SEQ ID NO: 14 | GGGGSGGGGSGGGGSGGGGS |

| TGFβ1 | SEQ ID NO: 15 | ALDTNYCFSSTEKNCCVRQLYIDFRKDLGWKWIHEPKGYHANFCLGPCP YIWSLDTQYSKVLALYNQHNPGASAAPCCVPQALEPLPIVYYVGRKPKVE QLSNMIVRSCKCS |
|---|---|---|
| TGFβ2 | SEQ ID NO: 16 | ALDAAYCFRNVQDNCCLRPLYIDFKRDLGWKWIHEPKGYNANFCAGACP YLWSSDTQHSRVLSLYNTINPEASASPCCVSQDLEPLTILYYIGKTPKIEQL SNMIVKSCKCS |
| TGFβ3 | SEQ ID NO: 17 | ALDTNYCFSSTEKNCCVRQLYIDFRKDLGWKWIHEPKGYHANFCLGPCP YIWSLDTQYSKVLALYNQHNPGASAAPCCVPQALEPLPIVYYVGRKPKVE QLSNMIVRSCKCS |

**FIG. 8 Cont.**

Europäisches
Patentamt

European
Patent Office

Office européen
des brevets

# EUROPEAN SEARCH REPORT

Application Number

EP 24 15 0099

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE CRESCENZO G ET AL: "Three Key Residues Underlie the Differential Affinity of the TGF@b Isoforms for the TGF@b Type II Receptor", JOURNAL OF MOLECULAR BIOLOGY, ACADEMIC PRESS, UNITED KINGDOM, vol. 355, no. 1, 6 January 2006 (2006-01-06), pages 47-62, XP024950470, ISSN: 0022-2836, DOI: 10.1016/J.JMB.2005.10.022 [retrieved on 2006-01-06] * tables 1, 2 * | 1-15 | INV. C07K14/71 C07K14/495 A61K38/17 |
| X | WO 2008/157367 A1 (GENZYME CORP [US]; QIU HUAWEI [US] ET AL.) 24 December 2008 (2008-12-24) * sequence 2 * * figure 1 * | 1-4,8 | |
| X | WO 2021/081258 A1 (CUE BIOPHARMA INC [US]) 29 April 2021 (2021-04-29) * paragraph [0306] * | 1-4,8 | TECHNICAL FIELDS SEARCHED (IPC) C07K A61K |

The present search report has been drawn up for all claims

| Place of search | Date of completion of the search | Examiner |
|---|---|---|
| The Hague | 16 May 2024 | Schmitz, Till |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 15 0099

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2024

| Patent document cited in search report | | Publication date | Patent family member(s) | | Publication date |
|---|---|---|---|---|---|
| WO 2008157367 | A1 | 24-12-2008 | EP | 2171062 A1 | 07-04-2010 |
| | | | JP | 2010529859 A | 02-09-2010 |
| | | | US | 2010204104 A1 | 12-08-2010 |
| | | | WO | 2008157367 A1 | 24-12-2008 |
| WO 2021081258 | A1 | 29-04-2021 | AU | 2020372413 A1 | 09-06-2022 |
| | | | BR | 112022007749 A2 | 05-07-2022 |
| | | | BR | 122023021925 A2 | 27-02-2024 |
| | | | CA | 3155510 A1 | 29-04-2021 |
| | | | CN | 114728072 A | 08-07-2022 |
| | | | EP | 4034170 A1 | 03-08-2022 |
| | | | IL | 292253 A | 01-06-2022 |
| | | | JP | 2023500066 A | 04-01-2023 |
| | | | KR | 20220087491 A | 24-06-2022 |
| | | | TW | 202130655 A | 16-08-2021 |
| | | | US | 2022372093 A1 | 24-11-2022 |
| | | | US | 2023416322 A1 | 28-12-2023 |
| | | | WO | 2021081258 A1 | 29-04-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63436822

- WO 2008077546 A

**Non-patent literature cited in the description**

- **CHOWDHURY ; SANJIB ; SUDHAKAR AMMANAMANCHI ; GILLIAN M. HOWELL.** Epigenetic targeting of transforming growth factor β receptor II and implications for cancer therapy. *Molecular and cellular pharmacology,* 2009, vol. 1.1, 57
- **BIERIE, B et al.** TGFβ: the molecular Jekyll and Hyde of cancer. *Nature Reviews Cancer,* 2006, vol. 6.7, 506-520
- **KIM, B et al.** Novel therapies emerging in oncology to target the TGF-β pathway. *Journal of Hematology & Oncology,* 2021, vol. 14.1, 1-20

- **LIND, H. et al.** Dual targeting of TGF-β and PD-L1 via a bifunctional anti-PD-L1/TGF-βRII agent: status of preclinical and clinical advances. *Journal for immunotherapy of cancer,* 2020, vol. 8.1
- **DAVIS et al.** *Basic Methods In Molecular Biology,* 1986
- **DHANDA et al.** Development of a strategy and computational application to select candidate protein analogues with reduced HLA binding and immunogenicity. *Immunology,* 2018, vol. 153.1, 118-132